## Europäisches Patentamt

## European Patent Office

## Office européen des brevets

(19)

(11) Veröffentlichungsnummer: **0 000 773**
**B1**

(12)

# EUROPÄISCHE PATENTSCHRIFT

(45) Veröffentlichungstag der Patentschrift:
**28.01.81**

(21) Anmeldenummer: **78100585.5**

(22) Anmeldetag: **03.08.78**

(51) Int. Cl.³: **A 61 L 2/10**, C 02 F 1/32,
B 01 J 19/08, A 23 L 3/28

(54) **Mehrkammer-Photoreaktor, Mehrkammer-Bestrahlungsverfahren.**

(30) Priorität: **06.08.77 DE 2735550**

(43) Veröffentlichungstag der Anmeldung:
**21.02.79 Patentblatt 79/4**

(45) Bekanntmachung des Hinweises auf die Patenterteilung:
**28.01.81 Patentblatt 81/4**

(84) Benannte Vertragsstaaten:
**BE CH DE FR GB LU NL SE**

(73) Patentinhaber: **Schenck, Günther Otto, Prof. Dr.,
Bismarckstrasse 31, D-4330 Mülheim-Ruhr (DE)**

(72) Erfinder: **Schenck, Günther Otto, Prof. Dr.,
Bismarckstrasse 31, D-4330 Mülheim-Ruhr (DE)**

(74) Vertreter: **Wolgast, Rudolf, Dr. et al,
Bökenbusch 41 Postfach 11 03 86,
D-5620 Velbert 11 Langenberg (DE)**

(56) Entgegenhaltungen:
**DE-A-2 108 062**
**FR-A-2 308 409**
**US-A-3 637 342**
**US-A-4 012 321**

EP 0 000 773 B1

ACTORUM AG.

## Mehrkammer-Photoreaktor, Mehrkammer-Bestrahlungsverfahren

Die Erfindung betrifft ein Verfahren zur Reinigung, insbesondere zur Entkeimung und Desinfektion unter Verwendung einer Strahlungsquelle für ultraviolette Strahlung im Wellenlängenbereich von 240 bis 320 nm, bei dem ein fliessfähiges Medium zur Einhaltung einer vorbestimmten Mindestdosis der ultravioletten Strahlung mit einem bestimmten Durchfluss durch einen Durchflussreaktor, der durch mindestens eine für ultraviolette Strahlung durchlässige Trennwand in mindestens zwei Bestrahlungskammern senkrecht zur Durchstrahlungsrichtung unterteilt ist, gefördert wird, und bei dem in den in bezug auf die durch die Strahlungsquelle bestimmte Durchstrahlungsrichtung hintereinander angeordneten Bestrahlungskammern durch das Medium in allen Bestrahlungskammern ein bestimmter Anteil und in der der Strahlungsquelle unmittelbar benachbarten Bestrahlungskammer ein Teil der in den Durchflussreaktor eintretenden ultravioletten Strahlung absorbiert wird. Die Erfindung betrifft auch eine Vorrichtung zur Ausübung des Verfahrens nach einem der vorstehenden Ansprüche, bestehend aus einer Strahlungsquelle mit mindestens einem Strahler, der ultraviolette Strahlung im Wellenlängenbereich von 240 bis 320 nm emittiert, aus einem durch mindestens eine für die ultraviolette Strahlung durchlässige Trennwand in mindestens zwei Bestrahlungskammern unterteilten Durchflussreaktor mit einer Zuleitung und einer Ableitung für das zu bestrahlende Medium, dessen Bestrahlungskammern in bezug auf die durch die Strahlungsquelle bestimmte Durchstrahlungsrichtung hintereinander angeordnet sind, wobei in allen Bestrahlungskammern ein bestimmter Anteil und in der der Strahlungsquelle unmittelbar benachbarten Bestrahlungskammer ein Teil der in den Durchflussreaktor eintretenden ultravioletten Strahlung durch das Medium absorbierbar ist, und aus einer Durchflusssteuereinrichtung zur Einstellung eines bestimmten Durchflusses zwecks Einhaltung einer vorbestimmten Mindestdosis der ultravioletten Strahlung.

Verfahren und Vorrichtungen zur Reinigung, insbesondere zur Entkeimung oder Desinfektion durch ultraviolette Strahlen werden mit Vorteil anstelle chemischer Mittel eingesetzt, um pathogene, toxische oder anderweitig störende und gegen ultraviolette Strahlen empfindliche Bestandteile aus Wasser zu entfernen. Dabei kann es sich um Mikroorganismen wie Bakterien, Sporen, Hefen, Pilze oder Algen, aber auch um Viren oder Bakteriophagen handeln. Auch kann es sich um solche die Umwelt belastende Verunreinigungen handeln wie cancerogene Aromaten, mannigfaltige Halogen-, vor allem Chlorverbindungen, z.B. Chlorphenole. Die Bestrahlung kann bei der Trinkwasseraufbereitung eingesetzt werden und ist besonders nützlich in Verbindung mit Ionenaustauscher- oder Umkehr-Osmose-Anlagen. Sie kann auch Schwimmbadwasser auf Trinkwasserqualität desinfizieren. Das UV-Bestrahlungsverfahren kann aber auch für Umlaufwasser beispielsweise von Klimaanlagen in Krankenhäusern eingesetzt werden und kann zu wesentlich höheren Entkeimungsgraden führen als sie für Trinkwasser verlangt werden, was z.B. für den Einsatz in ophthalmologischen Präparaten oder bei der Verwendung als Spülmittel im Operationssaal eine Voraussetzung ist. Weitere Einsatzbereiche finden sich z.B. in der Brauerei- und Getränkeindustrie, in der Nahrungsmittel-, Pharma- und Kosmetika-Industrie, bei der Reinigung von Abwässern oder der Herstellung reinsten Meerwassers für biotechnische Zwecke.

Photochemische Entkeimungs- bzw. Desinfektions- und Entgiftungsreaktionen folgen den bekannten Grundprinzipien photochemischer Reaktionen. Im allgemeinen ist die Konzentration der pathogenen und sonstigen durch die UV-Bestrahlung zu entfernenden Verunreinigungen sehr niedrig. Praktisch wird daher die Absorption des zu bestrahlenden Mediums durch andere Inhaltsstoffe bestimmt, deren Absorption mit der der Mikroorganismen etc. konkurriert. Dabei ist eine möglichst hohe Ausnutzung des verfügbaren Photonenstroms anzustreben. Hierzu genügen im allgemeinen solche Schichtdicken, in denen 90% der eingestrahlten Photonen absorbiert werden, da bei einer Verdoppelung dieser Schichtdicke nur weitere 9% der eingestrahlten Photonen zusätzlich absorbiert werden können. In der UV-Entkeimungstechnik bezeichnet man daher die durch 90% Absorption charakterisierte Schichtdicke als «wirksame Eindringtiefe». Diese kann bei einer Wellenlänge von 254 nm viele Dezimeter in besonders reinem Wasser, aber auch nur Bruchteile von Millimetern in Milch betragen.

Führt man die Ultraviolett-Bestrahlung bis zu einem Umsatz (Inaktivierung) von 90 bis 99% der anfänglich vorhandenen Mikroorganismen bzw. Verunreinigungen durch, so zeigt sich angenähert ein exponentieller Verlauf wie bei kinetisch analogen photochemischen Reaktionen. Der vorgenannte Umsatz von 90 bis 99% erfolgt dabei in einem Bruchteil der Zeit, die für Entkeimungs- bzw. Entgiftungsreaktionen im allgemeinen erforderlich ist. Hier interessiert dann nicht mehr die absolute Höhe des erzielten Umsatzes, der sich asymptotisch der Eingangszahl (Anzahl Keime/Volumen) nähert. Vielmehr interessiert nun nur noch die Menge an gereinigtem Medium eines verlangten Reinigungsgrades. Hier zeigt sich, dass das durch photochemische Überlegungen nahegelegte Arbeiten mit einer Schichtdicke entsprechend 90% Absorption, also mit der sog. «wirksamen Eindringtiefe», kein optimales Ergebnis liefert. Infolge des exponentiellen Lambertschen Absorptionsgesetzes kommt es in der durchstrahlten Schicht zu einer inhomogenen Geschwindigkeitsverteilung der Reinigung. Wegen der bei den heute verwendeten leistungsfähigen Strahlungsquellen in den Durchflussreaktoren überwiegend laminaren Strömungscharakteristik des durchstrahlten Mediums kommt es in diesem zum Aufbau einer logarithmischen Verteilung der Reinigungsgrade, wobei die wesentlich geringere Reinigung in grösserer Entfernung von der Strahlungsquelle überwiegt.

Es ist ein Photoreaktor mit annähernd parallel gerichteter Einstrahlung bekannt, bei dem die Strahlungsquelle oberhalb der Obefläche des zu bestrahlenden Mediums in einem Reflektor angeordnet ist (M. Luckiesh, Applications of germicidal, erythemal and infrared energy, Van Nostrand, New York, 1946. S. 257–265; Firmenschrift «Germicidal lamps and applications», LS-179, General Electric Company). Photoreaktoren dieser Art sind jedoch nur im Zusammenhang mit frei fliessenden Medien verwendbar, nicht aber im Zusammenhang mit Drucksystemen, in denen das zu bestrahlende Medium unter Druck durch den Photoreaktor gefördert wird. Für solche Vorrichtungen ist vorgeschlagen worden, den Photoreaktor ringförmig auszubilden und die Strahlungsquelle im Innenraum des Ringes unterzubringen; dabei ist als Strahlungsquelle eine Quecksilberhochdrucklampe (W. Buch, Wasserentkeimungsgerät «Uster», AEG-Mitteilungen 1936, Nr. 5, S. 178–181), aber auch eine Quecksilberniederdrucklampe (K. Wuhrmann, «Desinfektion von Wasser mittels Ultraviolett-Bestrahlung», Gas/Wasser/Wärme 1960, Bd. 14, S. 100–102) bzw. Bündel von Quecksilberniederdrucklampen verwendet worden (P. Ueberall, «Die chemikalienfreie Trink- und Brauchwasserentkeimung mit ultravioletten Strahlen», Die Stärke 1969, Bd. 21, S. 321–327). Zum Ausgleich der durch das Lambertsche Absorptionsgesetz und die Geometrie des Photoreaktors bedingten starken Abnahme der Bestrahlungsstärke in dem Photoreaktor ist vorgeschlagen worden, den Photoreaktor aus einer oder mehreren parallelen Bestrahlungskammern aufzubauen, die von mehreren in Reflektoren angeordneten UV-Strahlern umgeben sind, wobei zusätzlich innen weitere UV-Strahler vorgesehen werden können (DE-OS 21 19 961, 22 05 598; FR-OS 23 08 409). Zu den Photoreaktoren mit einer Strahlungsquelle mit radialer Ausstrahlung gehören auch noch solche, deren Strahler einfach oder mehrfach nach Art einer Tauchlampe in einem geeigneten durchströmten Behälter untergebracht ist (L. Grün, M. Pitz, «UV-Strahlen in Düsenkammern und Luftkanälen von Klimaanlagen in Krankenhäusern», Zbl. für Hygiene, I. Abteilung Orig. 1974, Bd. B 159, S. 50–60).

Obwohl die effektiven Eindringtiefen für 90% Absorption für viele Medien bekannt sind, weisen die bekannten Photoreaktoren im allgemeinen soche Schichtdicken auf, die nur Bruchteile der effektiven Eindringtiefe ausmachen.

Es sind auch Mehrkammer-Photoreaktoren bekannt, bei denen das zu bestrahlende Medium hintereinander durch mehrere Bestrahlungskammern gefördert wird, die einer Strahlungsquelle zugeordnet sind. Bei einem solchen Mehrkammer-Photoreaktor ist eine mittlere Bestrahlungskammer, die eine Strahlungsquelle aus mehreren parallelen UV-Strahlern enthält, von seitlichen, im Querschnitt segmentförmigen Bestrahlungskammern umgeben; zwischen der mittleren und den seitlichen Bestrahlungskammern befinden sich UV-durchlässige Trennwände. Die UV-Strahler sind so angeordnet und die Schichtdikken der Bestrahlungskammern sind so bemessen, dass praktisch die gesamte UV-Strahlung allein von dem zu bestrahlenden Medium und nicht an der Innenfläche der Aussenwand des Reaktors absorbiert wird (US-PS 3 637 342).

Zur Erzielung höherer Wirksamkeit und Leistung in der Desinfektion ist vorgeschlagen worden, einen annularen Photoreaktor mit einer Gesamtschichtdicke entsprechend einer Gesamtabsorption von mindestens 90% so zu unterteilen, dass die Intensität der UV-Strahlung an der Unterteilungsstelle mindestens gleich dem Mittelwert der Intensität im Photoreaktor ist (US-PS 2 669 661). Auf die Art der Unterteilung kommt es dabei nicht an, und der Durchfluss durch den Photoreaktor wird so eingestellt, dass an der Unterteilungsstelle eine Abtötung von 99% erreicht wird. Die angestrebte Optimierung wird dadurch jedoch nicht erzielt, denn die Konstruktion und Betriebsweise dieses Photoreaktors ist gerade bei Gesamtabsorptionen über 90% ungünstig.

Bestrahlt man (bei paralleler Einstrahlung) in einer Schicht mit 90% Absorption und mit einer so hohen Dosis, dass die Entkeimung in der ersten Schicht mit 10% Absorption mindestens $10^{-10}$ erreicht, so ergibt sich eine Inhomogenität der Entkeimungsgrade, die von $10^{-9}$ in der ersten Schicht bis zu $10^{-1}$ in der letzten Schicht reicht. Als mittleres Ergebnis wird dann ein Entkeimungsgrad von der Grössenordnung $10^{-2}$ erreicht, was wenig befriedigt, wenn man berücksichtigt, dass der theoretisch unter Annahme einer nicht logarithmisch abfallenden, sondern mittleren Bestrahlungsstärke ereichbare Entkeimungsgrad in der Grössenordnung von $10^{-4}$ liegt.

Die Aufgabe der vorliegenden Erfindung besteht demgemäss darin, ein Verfahren und eine Vorrichtung zu dessen Ausübung anzugeben, die eine optimale Ausnutzung der von der Strahlungsquelle ausgehenden UV-Strahlung bei möglichst hoher Leistung gestatten.

Erfindungsgemäss wird die Aufgabe in bezug auf das Verfahren dadurch gelöst, dass das Medium in allen Bestrahlungskammern insgesamt nicht mehr als $(1-0.5^n) \cdot 100\%$ der in den Durchflussreaktor eintretenden Strahlung absorbiert, wobei n die Zahl der Bestrahlungskammern ist. In bezug auf die Vorrichtung wird die Aufgabe dadurch gelöst, dass die von der Zahl n der Bestrahlungskammern und von der UV-Durchlässigkeit des zu bestrahlenden Mediums bestimmte Gesamtschichtdicke des Durchflussreaktors so bemessen ist, dass die Gesamtabsorption nicht mehr als $(1-0.5^n) \cdot 100\%$ der in den Durchflussreaktor eintretenden Strahlung liegt.

Die Erfindung geht aus von der Erkennntnis, dass bei einer Unterteilung des Photoreaktors die Schichtdicke der Bestrahlungskammern jeweils so gewählt werden kann, dass sich die Änderung der Bestrahlungsstärke in der Schichtdicke nicht zu ungünstig auf die Bestrahlungsökonomie auswirkt. Es wird dadurch in jeder Bestrahlungskammer eine weniger inhomogene Verteilung der Entkeimungsgrade erzielt. Bei einer Schichtdicke für 90% Absorption kann eine vier- bis fünffache Unterteilung dazu führen, dass die Unterschiede der Entkeimungsgrade innerhalb jeder Bestrahlungskammer weniger als 3 Grössenordnungen betragen, während die Unterschiede im nicht unterteilten Photoreaktor über 8 Grössenordnungen ausmachen. Das Prinzip beruht also darauf, dass man die mit zunehmender Schichtdicke durch ein Optimum gehende und dann wieder stark abnehmende Effizienz des Photoreaktors so einstellt, dass man mit einer Schicht nur teilweiser Absorption arbeitet und die diese Schicht verlassenden Photonen alsdann in folgenden Schichten ähnlicher oder gleicher, nur teilweiser Absorption ausnutzt. Die durch die Unterteilung erzielte günstige Wirkung ist weitgehend unabhängig von der Bestrahlungsgeometrie des jeweiligen Photoreaktors. Sie wird sowohl bei Photoreaktoren gefunden, bei denen die Strahlungsquelle von Tauchlampen gebildet wird, als auch bei ringförmigen Photoreaktoren, bei denen die Strahlungsquelle im Innenraum und/oder aussen angebracht ist; sie wird ebenfalls bei Photoreaktoren gefunden, deren Strahlungsquelle über der Oberfläche des Mediums angeordnet ist.

In der Praxis wird üblicherweise das optische Absorptionsverhalten eines Mediums durch die optische Durchlässigkeit, die Transmission, bei 1 cm Schichtdicke, abgekürzt T (1 cm), angegeben.

Im folgenden wird daher zum besseren Anschluss an diese Angabe unter «Absorption» die Summe aller lichtschwächenden Eigenschaften eines Mediums zusammengefasst und $-\ln T$ (1 cm) $= \frac{1}{\alpha}$ bzw. $-\log T$ (1 cm) $= \frac{1}{\varepsilon}$ gesetzt.

Die nähere Analyse hat gezeigt, dass die Bestrahlungsökonomie durch die Inhomogenität des Entkeimungsgrades in allen Schichten des bestrahlten Mediums negativ beeinflusst wird, besonders stark in denen, die der höchsten Bestrahlungsstärke ausgesetzt sind. Um einerseits so viel als möglich von der für die Wirksamkeit der Entkeimung besonders günstigen hohen Bestrahlungsstärke in unmittelbarer Nachbarschaft der Strahlungsquelle auszunutzen und andererseits durch die Inhomogenität in der Verteilung des Entkeimungsgrades so wenig wie möglich von dieser günstigen Wirkung einzubüssen, sollte daher die Absorption der ultravioletten Strahlung in der Bestrahlungskammer, die der Strahlungsquelle unmittelbar benachbart ist, einen bestimmten Höchstwert nicht überschreiten. Um auch die übrigen Bestrahlungskammern hinreichend zur Wirkung zu bringen, sollte die von deren Zahl und der UV-Durchlässigkeit des Mediums bestimmte Gesamtschichtdicke des Durchflussreaktors ebenfalls einen bestimmten Höchstwert nicht überschreiten. Die Absorption der ultravioletten Strahlung in der der Strahlungsquelle unmittelbar benachbarten Bestrahlungskammer sollte höchstens im Bereich von 50% der in den Durchflussreaktor eintretenden Strahlung liegen. Wie vorstehend bereits dargelegt wurde, wird die Effizienz der Reinigung bzw. Entkeimung durch den Gradienten der Bestrahlungsstärke zwischen Eintritt und Austritt der jeweiligen Bestrahlungskammer bestimmt. Das gilt beim Mehrkammer-Photoreaktor für jede einzelne Bestrahlungskammer, weshalb beispielsweise bei zwei Bestrahlungskammern die Gesamtabsorption der einfallenden Strahlung 75% nicht übersteigen sollte, um für jede Bestrahlungskammer diesen Gradienten ausreichend klein und die Effizienz insgesamt so gross wie möglich zu halten.

Zur Erhöhung des Entkeimungsgrades kann es vorteilhaft sein, dem Medium vor oder während der Bestrahlung ein Oxidationsmittel zuzuführen. Das Oxidationsmittel kann Sauerstoff, Ozon, Halogen oder ein Hypohalogenit sein.

Die Empfindlichkeit von Mikroorganismen gegen Ultraviolettstrahlung ist sehr unterschiedlich; beispielsweise ist die Empfindlichkeit von Pilzen oder Algen um mehr als 2 Grössenordnungen geringer als die Empfindlichkeit von Bakterien. Es ergibt sich daraus beim Einsatz von Durchflussreaktoren für die Entkeimung ein weiter Dosisbereich, der in seinem ganzen Umfang nicht einfach durch Erhöhung des Strahlungsflusses der Strahlungsquelle und/oder Verringerung des Durchflusses des zu bestrahlenden Mediums erfasst werden kann. Nach der Erfindung ist daher vorgesehen, dass mindestens ein Teilstrom des bestrahlten Mediums nach dem Durchlauf in den Durchflussreaktor zurückgeführt wird. Auf diese Weise wird das zu bestrahlende Medium mehrfach durch den Reaktor geführt und somit dem entsprechenden Vielfachen der Dosis des einfachen Durchlaufs bestrahlt. Dieses Verfahren empfiehlt sich auch in solchen Fällen, in denen das entkeimte Medim aus einer Ultraviolett-Entkeimungsanlage in wechselnden Mengen entnommen wird.

Nach dem erfindungsgemässen Verfahren wird das Medium vorteilhafterweise nacheinander durch die Bestrahlungskammern des Durchflussreaktors gefördert. Dadurch wird, wie bereits weiter oben erläutert wurde, die Effizienz der Reinigung bzw. Entkeimung beträchtlich erhöht. Der Durchflussreaktor kann dann mit einem grösseren Durchfluss betrieben werden, so dass die Strömungsgeschwindigkeiten in den Bestrahlungskammern des Mehrkammer-Photoreaktors gegenüber der Strömungsgeschwindigkeit im Einkammer-Photoreaktor erhöht sind. Durch eine solche Erhöhung der Strömungsgeschwindigkeiten und durch die Verringerung der Querschnitte der Bestrahlungskammern werden Strömungskurzschlüsse vermieden, die bei Einkammer-Photoreaktoren bei hohen Schichtdicken und geringen Strömungsgeschwindigkeiten auftreten. Es empfiehlt sich, beim Mehrkammer-Photoreaktor mit einer Strömungsgeschwindigkeit zu arbeiten, die an oder über der Grenze der Turbulenz des durchströmenden Mediums liegt. Auf diese Weise wird nicht nur die Bildung von Niederschlägen aus dem bestrahlten Medium im Mehrkammer-Photoreaktor wirksam unterdrückt, sondern darüber hinaus in der der Strahlungsquelle unmittelbar benachbarten Kammer des Mehrkammer-Photoreaktors ein besonders guter Wärmeübergang von der Strahlungsquelle auf das durchfliessende Medium erzielt, so dass Überhitzungen vermieden werden.

Die erwähnte beträchtliche Erhöhung der Effizienz des Durchflussreaktors durch die Unterteilung ist nicht daran gebunden, dass das Medium nacheinander durch die Bestrahlungskammern des Durchflussreaktors gefördert wird. Vielmehr ist dies eine charakteristische Eigenschaft des Mehrkammer-Photoreaktors. Leitet man nämlich das Medium parallel durch die Bestrahlungskammern, so lässt sich der Durchfluss durch jede einzelne Bestrahlungskammer so einstellen, dass in jeder Bestrahlungskammer die gleiche Mindestdosis verabreicht und somit der gleiche Entkeimungsgrad erzielt wird und die mit unterschiedlichen Strömungsgeschwindigkeiten fliessenden Anteile des Mediums nach dem Verlassen der Bestrahlungskammern wieder vereinigt werden können. Die parallele Flüssigkeitsführung ist zwar durch ihre apparative Ausrüstung aufwendiger, kann aber für die gleichzeitige Bestrahlung verschiedener Medien von Vorteil sein.

Im einfachsten Fall, beispielsweise bei der Photoentkeimung von Seewasser, besteht bei der erfindungsgemässen Vorrichtung der Durchflussreaktor aus einem trogartigen Gefäss, das durch eine aus Quarzglasscheiben gefertigte Trennwand in eine untere und eine obere Bestrahlungskammer unterteilt ist. Die Strahlungsquelle befindet sich oberhalb des trogartigen Gefässes in einem Reflektorsystem, das die von der Strahlungsquelle ausgehende Strahlung parallel in das trogartige Gefäss richtet. Das Seewasser tritt in die eine der beiden Kammern ein und durchsetzt nach Durchlauf durch die erste Kammer die zweite Kammer. Es kann bei einer solchen Anordnung auch das durch Quarzglasscheiben in Bestrahlungskammern unterteilte Gefäss selbst aus Quarzglas bestehen, wobei die Strahlungsquelle von paarweise an gegenüberliegenden Seiten des Gefässes in einem System von Einzelreflektoren angebrachten Strahlern gebildet wird.

Aus dem Bereich der Hospitalhygiene sind im Umlauf betriebene Entkeimungsanlagen mit einer Strahlungsquelle aus einem oder mehreren, jeweils in einem Hüllrohr nach Art einer Tauchlampe in einen Behälter eingesetzten Strahlern bekannt, beispielsweise bei Klimawäschern. Solche Anordnungen besitzen ungünstige Strömungsverhältnisse, die dazu führen, dass ein Teil des in dem Behälter befindlichen Wassers erheblich höhere Bestrahlungsdosen erhält als nötig, während ein grosser Teil des Behälterwassers zu niedrigen Dosen ausgesetzt bleibt. Es besteht daher die Gefahr, dass aus diesem Wasser Keime an die von der Klimaanlage umgewälzte Luft abgegeben werden. Nach der Erfindung ist deshalb vorgesehen, dass ggf. jedes Hüllrohr von wenigstens einem Quarzglasrohr unter Ausbildung wenigstens einer inneren Bestrahlungskammer umgeben ist und dass die inneren Bestrahlungskammern gemeinsam entweder eingangsseitig an die Zuleitung oder ausgangsseitig an die Ableitung des Durchflussreaktors angeschlossen sind. Im Gegensatz zu der bekannten Anordnung wird es in der erfindungsgemässen Vorrichtung sichergestellt, dass die Bestrahlung unabhängig von der Strömungsrichtung durch die inneren Bestrahlungskammern mit der gewünschten Mindestdosis erfolgt. Schliesst man dabei die inneren Bestrahlungskammern eingangsseitig an die Zuleitung an, so wird auf diese Weise das Bestrahlen in Gegenwart von Sauerstoff oder anderen Gasen erleichtert; schliesst man die inneren Bestrahlungskammern ausgangsseitig an die Ableitung des Durchflussreaktors an, so erhält man optimal entkeimtes Wasser an der Sprühdüse des Klimawäschers.

Nach einer Ausführungsform der Erfindung ist eine Vorrichtung, bei der die Strahlungsquelle und der Durchflussreaktor ringförmig zueinander angeordnet sind, so aufgebaut, das der Durchflussreaktor aus zwei mit Anschlussmitteln versehenen Verschlussteilen, die die Bestrahlungskammern stirnseitig begrenzen, und aus zwischen den Verschlussteilen an diesen angebrachten Rohrstücken unterschiedlichen Durchmessers besteht, die koaxial ineinander angeordnet sind und die Bestrahlungskammern längsseitig begrenzen. Dabei können die Verschlussteile für jede Bestrahlungskammer einen Anschlussstutzen besitzen, der über mindestens einen Innenkanal mit der zugehörigen Bestrahlungskammer in Verbindung steht. Es wird auf diese Weise ein aus koaxial zwischen den stirnseitigen Verschlussteilen gehalterten Quarzglasrohren bestehender ringförmiger Mehrkammer-Photoreaktor einfachen Aufbaus erhalten, bei dem die Bestrahlungskammern je nach den Anforderungen parallel- oder hintereinandergeschaltet werden können.

In der Serienschaltung stehen benachbarte Bestrahlungskammern an gegenüberliegenden Enden miteinander in Verbindung. Die besonderen Vorteile einer solchen Serienschaltung liegen darin, dass infolge der veränderten Strömungswege und -geschwindigkeiten eine günstigere Verteilung der eingestrahlten Energie auf das durchfliessende Medium und hierdurch eine wesentlich verbesserte Effizienz der angestrebten Reinigungs- bzw. Entkeimungsprozesse erreicht wird. Bei gleichem Durchfluss ist nämlich die mittlere Strömungsgeschwindigkeit in einem m-Kammer-Photoreaktor angenähert das m-fache der mittleren Strömungsgeschwindigkeit eines Einkammer-Photoreaktors. Im Mehrkammer-Photoreaktor durchläuft ein Volumteil des Mediums hintereinander sämtliche Bestrahlungskammern von der höchsten bis zur niedrigsten mittleren Bestrahlungsstärke oder vice versa, wodurch eine wesentlich gleichmässigere Verteilung der zugeführten Energie auf das durchströmende Medium erzielt wird. Dadurch wird eine Überbestrahlung im Nahbereich der Strahlungsquelle und ebenso eine Unterbestrahlung in entfernteren Bereichen vermieden.

Vorteilhafterweise sind bei der erfindungsgemässen Vorrichtung die Rohrstücke an ihren Enden abwechselnd abdichtend gehaltert und geführt, und benachbarte Bestrahlungskammern stehen jeweils an den geführten Enden der Rohrstücke miteinander in Verbindung. Dadurch wird eine Vereinfachung im Aufbau des Mehrkammer-Photoreaktors mit in Serie geschalteten Bestrahlungskammern erzielt, da sich die Verbindung zwischen den Bestrahlungskammern innerhalb des Durchflussreaktors befindet und in den Verschlussteilen nur noch Innenkanäle zu den Anschlusstutzen vorgesehen werden müssen, die als Eingangs- und Ausgangsanschluss dienen.

Mehrkammer-Photoreaktoren der vorstehend beschriebenen Art mit einem äusseren Quarzglasrohrstück können in bekannter Weise (DT-OS 2119961) konzentrisch von mehreren Strahlern umgeben sein, von denen jeder einen eigenen paraboloiden Reflektor aufweist, wodurch eine optimale Effizienz der Einstrahlung ermöglicht wird. Eine einwandfreie Funktion eines Einkammer-Photoreaktors dieser Art ist nur dann gewährleistet, wenn Kurzschlussphänomene bei der Durchströmung sicher vermieden werden.

Das innere Rohrstück kann bei der erfindungsgemässen Vorrichtung an beiden Enden durch entsprechende Durchbrüche der Verschlussteile hindurchgeführt und in den Durchbrüchen abdichtend gehalten sein. Dadurch wird die Anbringung einer Strahlungsquelle im Inneren des Mehrkammer-Photoreaktors ermöglicht, die zusätzlich zu den den Mehrkammer-Photoreaktor aussen umgebenden Strahlern vorgesehen sein kann. Dadurch werden die Strahlungsverluste, die beim Durchgang der Strahlung durch den Photoreaktor auftreten, in beträchtlichem Umfang kompensiert, und es wird bei entsprechender Abstimmung der Schichtdicke an die Transmissionsfaktoren des Mediums eine brauchbare Annäherung an eine gleich hohe Bestrahlungsstärke in allen Volumelementen des Photoreaktors erzielt.

Bei dem Mehrkammer-Photoreaktor nach der Erfindung kann das äussere Rohrstück strahlungsundurchlässig sein, eine Beobachtungsöffnung aufweisen und an die Verschlussteile abdichtend angeflanscht sein. Das ermöglicht einen stabileren und weiter vereinfachten Aufbau des Photoreaktors, in dessen Innerem die Strahlungsquelle angebracht ist. Zur Erhöhung der Bestrahlungsstärke in der Aussenkammer kann das äussere Rohrstück verspiegelt sein, vorzugsweise so, dass das durchfliessende Medium nicht auf die Verspiegelung einwirken kann.

Bei einem Photoreaktor mit an einem Verschlussteil abdichtend gehalterten Rohrstücken, deren inneres an dem dem Verschlussteil abgewandten Ende geschlossen und deren nach aussen folgendes an dem gleichen Ende offen ist, und mit mindestens einem an dem Verschlussteil angeordneten Anschluss, der über einen Innenkanal in dem Verschlussteil an die innere Bestrahlungskammer angeschlossen ist, kann erfindungsgemäss jedes der zweiten nach aussen folgenden, an dem einen Ende geschlossenen Rohrstücke nahe dem Verschlussteil mit Durchtrittsöffnungen versehen sein. Die einseitige Halterung der Rohrstücke kann Erleichterungen im Zusammenbau und in der Demontage des Mehrkammer-Photoreaktors bringen.

Vorteilhaft besitzt bei den erfindungsgemässen Mehrkammer-Photoreaktoren eine der Strahlungsquelle abgewandte Bestrahlungskammer eine Schichtdicke, die mindestens das Zweifache der Schichtdicke der der Strahlungsquelle unmittelbar benachbarten Bestrahlungskammer beträgt. Ein solcher Photoreaktor ist für alle Medien mit Transmissionsfaktoren im Bereich zwischen T (1 cm) = 0.6 und nahe 1 geeignet. Für die Entkeimung von Trinkwasser mit einem Transmissionsfaktor im niedrigen Bereich sind dann vorwiegend die beiden Bestrahlungskammern mit geringer Schichtdicke wirksam, während bei Trinkwasser mit hohem Transmissionsfaktor auch die der Strahlungsquelle abgewandte Bestrahlungskammer mit grösserer Schichtdicke mit guter Wirksamkeit einbezogen wird. Ein solcher Mehrkammer-Photoreaktor kann somit bei der Trinkwasserentkeimung im gesamten vorkommenden Bereich von Transmissionsfaktoren eingesetzt werden, ohne dass dazu zusätzliche Massnahmen in seinem Aufbau notwendig sind. Durch die Hinzunahme der Bestrahlungskammer mit grosser Schichtdicke bei Verwendung von Trinkwasser hoher Transmission ergibt sich eine hohe Leistung, die bei Photoreaktoren mit kleineren Schichtdicken bzw. bei einem Einkammer-Photoreaktor grösserer Gesamtschichtdicke nicht erzielbar ist.

Zweckmässig ist bei dem Mehrkammer-Photoreaktor nach der Erfindung eine Druckausgleichseinrichtung vorgesehen. Diese kann einen mit druckdichten Durchführungen versehenen, druckdicht mit dem die Rohrstücke halternden Verschlussteil verbundenen, an einen Barostaten angeschlossenen Deckel aufweisen, wobei der Sollwert der barostatischen Druckregelung vom Eingangsdruck des Mediums am Durchflussreaktor bestimmt ist. Durch eine solche Einrichtung wird der im Betrieb des Mehrkammer-Photoreaktors auf die aus Quarzglas bestehenden Rohrstücke einwirkende Druck ausgeglichen. Dadurch wird vermieden, dass an den spannungsempfindlichen Quarzglasrohren mechanische Spannungen auftreten, die zum Bruch führen könnten.

Eine Ausführung der erfindungsgemässen Vorrichtung, bei der ein Teilstrom des bestrahlten Mediums nach dem Durchlauf in den Durchflussreaktor zurückgeführt wird, ist dadurch gekennzeichnet, dass der Durchflussreaktor ausgangsseitig mit einem Strömungsteiler versehen ist, dessen einer Ausgang an die Entnahmeleitung und dessen zweiter Ausgang unter Zwischenschaltung einer Rücklauf-Förderpumpe und eines Rückschlagventils an den Eingang des Durchflussreaktors angeschlossen ist. Dabei kann die Rücklauf-Förderpumpe in ihrer Förderleistung einstellbar sein, um eine Änderung des Rücklaufverhältnisses herbeizuführen; es kann aber auch die Rücklaufleitung eine einstellbare Strömungsdrossel aufweisen. Mit einer solchen Vorrichtung können besonders hohe Entkeimungs- bzw. Reinigungsgrade erzielt werden; ausserdem ist sie für solche Anwendungsfälle geeignet, bei denen keine kontinuierliche Entnahme erfolgt.

Bei einer Ausführungsart der Vorrichtung nach der Erfindung ist die Strahlungsquelle von mindestens einer antimondotierten Xenon-Hochdrucklampe, die eine starke Emission im Wellenlängenbereich von 260 bis 280 nm besitzt, gebildet. Eine solche Lampe besitzt pro cm Emissionslänge eine bakterizide Dosisleistung, die mindestens um eine Grössenordnung höher ist als die entsprechende Strahlungsleistung üblicher Quecksilberniederdruckquarzlampen. Bei gleicher Strömungsgeschwindigkeit ist es daher möglich, den Dosisbereich um über eine Grössenordnung zu erhöhen; für die heutigen Bedürfnisse der Trinkwasserentkeimung ergibt sich somit, dass mit einer Strahlungsquelle aus antimondotierten Xenon-Hochdrucklampen sehr viel höhere Raum-Zeit-Ausbeuten erzielt werden können, als dies bisher möglich war. Neben dieser erheblichen Verbesserung der Leistungsfähigkeit beruht ein weiterer Vorteil des Einsatzes der antimondotierten Xenon-Hochdrucklampen darauf, dass wegen der minimalen Flüchtigkeit und Toxizität des Antimons die Möglichkeit einer gefährlichen Umweltverschmutzung bei einem Bruch der Lampe wesentlich geringer ist als bei den sonst üblichen Quecksilberdampflampen.

Für Anwendungsfälle, in denen ein möglichst breiter Bereich von UV-Strahlung des wirksamen Wellenlängenbereichs für die Reinigung, Entkeimung und/oder Desinfektion mittels ultravioletter Strahlung verwendet werden soll, kann es nützlich sein, dass die Strahlungsquelle zusätzlich zu der antimondotierten Xenon-Hochdrucklampe mindestens eine Quecksilberdampflampe aufweist.

Zur Erhöhung des von der Strahlungsquelle ausgehenden Strahlungsflusses pro Längeneinheit des Durchflussreaktors kann es zweckmässig sein, dass die Strahlungsquelle mindestens einen gewendelten Strahler enthält.

Bei ringförmiger Ausbildung des Durchflussreaktors kann die Strahlungsquelle im Inneren des Durchflussreaktors in achsnaher Stellung angeordnet sein. Eine solche Anordnung der Strahlungsquelle bewirkt die beste Strahlungsverteilung in radialer Richtung. Die Strahlungsquelle kann dabei unabhängig vom Durchflussreaktor gehaltert sein; bei anderen Ausführungen, z.B. bei einem Druckdurchflussreaktor, ist dagegen die Strahlungsquelle im Durchflussreaktor gehaltert. Die Anordnung der Strahlungsquelle im Inneren des Durchflussreaktors ist für Durchflussreaktoren kleineren Volumens vorzuziehen.

Bei einer Ausbildung eines ringförmigen Durchflussreaktors nach der Erfindung kann die Strahlungsquelle mindestens 4 achsparallel und symmetrisch zwischen dem Durchflussreaktor und einem diesen umgebenden Reflektorsystem angeordnete Strahler aufweisen. Dabei befindet sich zweckmässigerweise jeder Strahler in einem gesonderten, vorzugsweise paraboloiden Reflektor des Reflektorsystems, um eine optimale optische Effizienz der Einstrahlung in den Durchflussreaktor zu gewährleisten. Bei einer solchen Anordnung der Strahlungsquelle wird eine gleichmässigere Verteilung der Strahlung über das Gesamtvolumen des Reaktors erzielt als bei Anordnung der Strahlungsquelle im Inneren des ringförmigen Reaktors; sie ist für grossvolumige Durchflussreaktoren vorzüglich geeignet.

Eine weitere Verbesserung der Strahlungsverteilung kann in dem erfindungsgemässen Durchflussreaktor dadurch erzielt werden, dass ein Teil der die Strahlungsquelle bildenden Strahler im Inneren des Durchflussreaktors und ein anderer Teil der Strahler, mindestens 4, achsparallel und symmetrisch zwischen dem Durchflussreaktor und einem diesen umgebenden Reflektorsystem angeordnet sind, wobei sich die Strahler, wie vorstehend beschrieben, in gesonderten Reflektoren befinden.

Bei Vorrichtungen der vorstehend beschriebenen Art kann es zweckmässig sein, eine antimondotierte Xenon-Hochdrucklampe mindestens im Inneren des Durchflussreaktors anzuordnen; dadurch werden die bei Reflektoren unvermeidlichen Reflexionsverluste vermieden und die von der hochwirksamen antimondotierten Xenon-Hochdrucklampe ausgehenden Strahlen besser ausgenutzt; zugleich wird für diesen Strahler eine besondere Wasserkühlung überflüssig.

Die vorstehend beschriebenen Vorrichtungen zur Durchführung einer Reinigung, Entkeimung oder Desinfektion fliessfähiger Medien im Durchfluss mittels ultravioletter Strahlung erfordern zur Einhaltung einer vorbestimmten Mindestdosis der ultravioletten Strahlung Durchflussteuermittel, durch die sichergestellt wird, dass das die Bestrahlungskammern durchsetzende Medium in jedem Fall mit der geforderten Mindestdosis bestrahlt wird. Die erfindungsgemässen Durchflussteuermittel können im einfachsten Fall eine Strömungsdrossel, vorzugsweise eine einstellbare Strömungsdrossel, aufweisen. Bei konstantem Druck am Eingang oder Ausgang des Durchflussreaktors lässt sich der Durchfluss auf den jeweils erforderlichen Wert einstellen. Die Strömungsdrossel kann aus einer Engstelle in der Zuleitung oder Ableitung des Durchflussreaktors bestehen, sie kann aber auch durch ein einstellbares und in seiner Einstellung zeitlich unveränderliches Ventil gebildet sein.

Die erfindungsgemässen Durchflussteuermittel können aber auch einen vom Eingangsdruck unabhängigen Durchflussbegrenzer aufweisen. Solche Durchflussbegrenzer sind bekannt, und ihre Anwendung im Zusammenhang mit den hier beschriebenen Durchflussreaktoren ist besonders vorteilhaft, weil sie in jedem Fall verhindern, dass ein vorgegebener Durchfluss überschritten wird. Ein solches Überschreiten des vorgegebenen Durchflusses muss besonders bei den Durchflussreaktoren zur Photoentkeimung vermieden werden, da eine Erhöhung des Durchflusses notwendig zu einem Unterschreiten der vorbestimmten Mindestdosis führen muss.

Nach der Erfindung können die Durchflusssteuermittel auch eine Pumpe mit einstellbarer Förderleistung aufweisen. Eine solche Pumpe gestattet im weitesten Umfang die Anpassung des Durchflusses an die jeweils gewünschten Bestrahlungsdosen.

Erfindungsgemäss kann eine Steuereinrichtung für die Pumpe mit einstellbarer Förderleistung vorgesehen werden, die mit einem von der Überwachungseinrichtung mit Sollwerteinstellung ausgehenden Steuersignal beaufschlagt ist. Dabei kann die Steuereinrichtung einen Leistungsverstärker und einen von dem Pumpenmotor angetriebenen Tachogenerator aufweisen und das Tachogeneratorsignal kann dem Steuersignal der Überwachungseinrichtung am Eingang des Leistungsverstärkers entgegengeschaltet sein. Bekannte Überwachungseinrichtungen für Durchflussreaktoren für die Photoentkeimung enthalten einen Strahlungsdetektor, der an dem Durchflussreaktor angeordnet ist und auf die durch den Durchflussreaktor hindurchtretende Strahlung anspricht. Bei Unterschreiten eines voreingestellten Sollwertes wird von dem Detektor ein Signal abgegeben, durch das ein Ventil angesteuert wird, mittels dessen das zu bestrahlende Medium auf einen zweiten Durchflussreaktor gegeben wird, durch das ein Alarmsignal ausgelöst wird und durch das eine Reinigungsvorrichtung für den ersten Photoreaktor betätigt werden kann (US-PS 3 182 193). Es ist weiter eine Überwachungseinrichtung bekannt (US-PS 3 462 597), die bei Ausfall der Lampe, des Lampentransformators oder bei einem unzulässig grossen Absinken der Netzspannung ein Magnetventil in der Zuführung für das zu bestrahlende Medium schliesst. Diese bekannten Überwachungseinrichtungen sind aber lediglich dazu geeignet und benutzt worden, um in Notfällen den Betrieb des Durchflussreaktors sofort und unter Abgabe eines Notsignals zu unterbrechen bzw. auf einen zweiten Reaktor umzuschalten. Die erfindungsgemässe Einrichtung verbindet dagegen die Pumpe mit einstellbarer Förderleistung mit einer Steuereinrichtung, deren Ausgangssignal von der jeweils an der Überwachungseinrichtung gemessenen Strahlungsintensität abhängig ist. Auf diese Weise wird ermöglicht, den Durchfluss an die jeweilige Bestrahlungsstärke anzupassen. Das bedeutet, dass bei abnehmender Bestrahlungsleistung die Durchflussleistung im gleichen Verhältnis abnimmt, so dass sichergestellt ist, dass die Reinigungs- bzw. Entkeimungsqualität erhalten bleibt. Durch eine solche Steuereinrichtung können auf einfache Weise die Einflüsse der Alterung auf die Ausstrahlung der Strahlungsquelle berücksichtigt werden. Die geschilderte Steuerung des Durchflusses bei abnehmender Bestrahlungsstärke zur Erhaltung der eingestellten Dosis erlaubt einen besonders ökonomischen Bestrahlungsbetrieb im paarweisen Verbund der Mehrkammer-Photoreaktoren. Dabei wird der eine Photoreaktor mit einem neuen Lampensatz in Betrieb genommen, während der zweite seinen Betrieb während der halben Lebensdauer seiner Lampen fortsetzt. Dadurch werden beide entsprechend den jeweiligen Lampenleistungen optimal betrieben, und die Gesamtleistungsschwankung infolge der Alterung beträgt nur noch die Hälfte der bisherigen Grösse. Zugleich ist aber auch eine bessere Lampen- und Stromausnutzung gewährleistet und zugleich eine bessere Apparateausnutzung erreicht.

Ausführungsbeispiele der erfindungsgemässen Vorrichtung sind in den Abbildungen dargestellt und werden nachfolgend im einzelnen erläutert und beschrieben. Es zeigen

Fig. 1 eine perspektivische Ansicht eines ersten Ausführungsbeispiels des erfindungsgemässen Mehrkammer-Photoreaktors;

Fig. 2 einen Längsschnitt durch eine Teilanordnung bei einem zweiten Ausführungsbeispiel des erfindungsgemässen Mehrkammer-Photoreaktors;

Fig. 2A einen entsprechenden Schnitt durch eine abgeänderte Ausführung der Teilanordnung nach Fig. 2;

Fig. 3 eine perspektivische Ansicht eines Details von Fig. 2;

Fig. 4 einen Längsschnitt durch eine weitere Ausbildung der Teilanordnung;

Fig. 4A einen entsprechenden Schnitt durch eine abgeänderte Ausführung der Teilanordnung nach Fig. 4;

Fig. 5 eine Ansicht eines Details bei der Teilanordnung nach Fig. 4;

Fig. 6 eine perspektivische Ansicht des zweiten Ausführungsbeispiels des erfindungsgemässen Mehrkammer-Photoreaktors;

Fig. 7 eine Draufsicht auf eine weitere Ausbildung des Mehrkammer-Photoreaktors nach Fig. 6;

Fig. 8 eine Schnittansicht eines Details bei dem Mehrkammer-Photoreaktor nach Fig. 7;

Fig. 9 einen Längsschnitt durch ein drittes Ausführungsbeispiel des erfindungsgemässen Mehrkammer-Photoreaktors;

Fig. 10 einen Teillängsschnitt durch einen Mehrkammer-Photoreaktor nach Fig. 9 mit einer Druckausgleichseinrichtung;

Fig. 11 einen Längsschnitt durch ein viertes Ausführungsbeispiel des erfindungsgemässen Mehrkammer-Photoreaktors;

Fig. 12 einen Längsschnitt durch ein fünftes Ausführungsbeispiel des erfindungsgemässen Mehrkammer-Photoreaktors;

Fig. 13 ein Fliessdiagramm für den Rücklaufbetrieb eines erfindungsgemässen Mehrkammer-Photoreaktors;

Fig. 13A ein Fliessdiagramm für einen modifizierten Rückflussbetrieb entsprechend Fig. 13;

Fig. 14 eine Detailansicht eines Bauteils in dem Fliessdiagramm nach Fig. 13; und

Fig. 15 ein Blockschaltbild eines Details der elektrischen Überwachungseinrichtung für den Betrieb eines erfindungsgemässen Durchflussreaktors;

Fig. 16 einen Längsschnitt durch ein für parallelen Durchfluss abgeändertes viertes Ausführungsbeispiel nach Fig. 11;

Fig. 17 einen Längsschnitt durch ein für parallelen Durchfluss abgeändertes fünftes Ausführungsbeispiel nach Fig. 12.

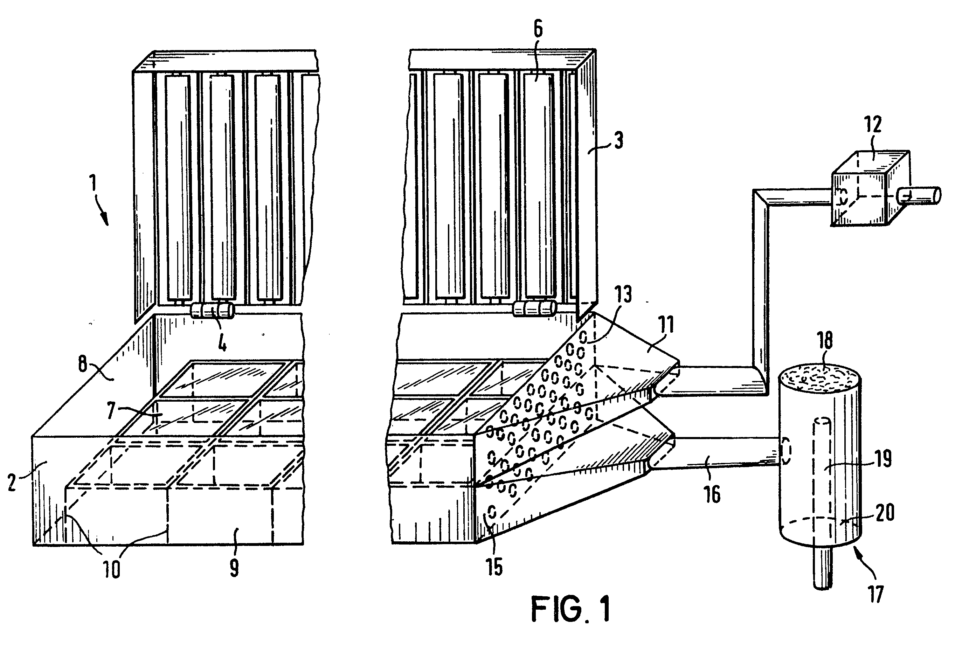

Fig. 1 zeigt einen Zweikammer-Photoreaktor 1 aus einem Durchflussreaktor in Gestalt eines trogartigen Gefässes 2 mit einem Deckel 3, der um Scharniere 4 schwenkbar an das trogartige Gefäss 2 angelenkt ist und durch einen Schnappverschluss in geschlossener Stellung gehalten wird. Das Gefäss 2 besteht aus Metall wie rostfreiem Stahl, kann aber auch aus jedem anderen UV-beständigen und sonstigen Anforderungen, z.B. lebensmittelrechtlichen Bestimmungen, genügendem Material (Steinzeug, emailliertes Blech etc.) gefertigt sein. Der Deckel 3 trägt innen eine Serie von zueinander parallelen paraboloiden Reflektoren mit einer besonders gut UVreflektierenden Oberfläche. Innerhalb der Reflektoren sind UV-Strahler 6 senkrecht zur Durchströmungsrichtung so angeordnet, dass der Strömungsquerschnitt des trogartigen Gefässes 2 unter Einschluss der Randbereiche gleichmässig bestrahlt wird. Für Zwecke der Entkeimung werden wassergekühlte, antimondotierte Xenon-Hochdrucklampen eingesetzt; alternativ eignen sich dafür auch Quecksilberniederdruck-Quarzlampen bekannter Bauart. Für die Reinigung in Anwesenheit oder Abwesenheit von Oxidationsmitteln kann man auch Quecksilberhochdrucklampen oder andere Strahler geeigneter Emissionsbereiche verwenden. Der Schnappverschluss ist mit einer Sicherheitsschaltung verbunden, durch die die Strahler 6 bei Öffnung des Schnappverschlusses automatisch abgeschaltet werden. Das trogartige Gefäss 2 ist in Strömungsrichtung durch Quarzglasscheiben 7 in zwei Bestrahlungskammern 8 und 9 unterteilt; die Bestrahlungskammer 9 ist als untere Bestrahlungskammer durch die Quarzglasscheiben 7 auf eine fixe Schichtdicke von 2 cm begrenzt, während die Schichtdicke des Mediums in der Bestrahlungskammer 8 mit Hilfe des weiter unten beschriebenen Niveaureglers 17 variiert werden kann. Die Quarzglasscheiben 7 sind auf einem herausnehmbaren Strebrahmen 10 aus rostfreiem Stahl gelagert; die Quarzglasscheiben 7 sind an dem Strebrahmen 10 und dieser selbst ist an der Innenwandung des trogartigen Gefässes 2 mittels eines gegen UV-Strahlung beständigen Kitts abdichtend befestigt. Anstelle der Verkittung kann die Abdichtung auch durch vorgeformte und UV-beständige Dichtungen erfolgen. Die Bestrahlungskammern 8, 9 kommunizieren an ihrem dem Ein- und Ausgang des trogartigen Gefässes 2 abgewandten Ende miteinander. Die obere Bestrahlungskammer 8 ist über eine Zuleitung 11 an einen Durchflussbegrenzer 12 angeschlossen. Der Durchflussbegrenzer dient dazu, den Durchfluss auch bei Erhöhung des Eingangsdrucks auf den zulässigen Maximalwert zu begrenzen; solche Durchflussbegrenzer werden beispielsweise von der Firma Eaton Corp., Controls Division, 191 East North Ave. Carol Stream, Illinois 60 187, USA, vertrieben. Die Zuleitung 11 mündet in die Bestrahlungskammer 8 über eine Lochplatte 13, die ein Ausgleichselement für das Strömungsprofil darstellt und sich über die gesamte Breite der Bestrahlungskammer 8 erstreckt. Die Bestrahlungskammer 9 mündet über eine gleichartige Lochplatte 15, die ebenfalls als Ausgleichselement für das Strömungsprofil wirkt, in eine Ableitung 16 mit einem Niveauregler 17, der zum Schutz gegen Verunreinigungen eine luftdurchlässige Abdeckung 18, z.B. aus Watte, trägt.

Die Lochplatten 13, 15 bestehen aus Material, das gegen UV-Strahlung und gegen das durchströmende Medium beständig ist und selbst keine störenden Verunreinigungen an das durchströmende Medium abgibt (rostfreier Stahl, beschichtete Metalle, Kunststoff, Keramik, Quarz, Glas). Die Weite der Löcher ist so gross, dass die Strömung nicht wesentlich behindert ist, aber doch ein über die Durchtrittsfläche gleichmässiges Strömungsprofil erzeugt wird. Zu dem gleichen Zwecke können die Löcher auch durch Öffnungen anderer Gestalt wie Schlitze ersetzt werden. Die Lochplatten 13, 15 sind mit dem trogartigen Gefäss 2 einerseits und dem Übergangsstück der Zuleitung 11 bzw. der Ableitung 16 andererseits in geeigneter Weise abdichtend verkittet.

Der Niveauregler 17 besitzt ein Innenrohr 19, das abgedichtet in einem Überlaufgefäss 20 vertikal verschiebbar geführt ist und den Auslauf des trogartigen Gefässes 2 bildet. Durch Vertikalverschiebung des Innenrohres 19 in dem Niveauregler 17 können in Anpassung an die optische Dichte des durch die Zuleitung 11 in den Durchflussreaktor eintretenden Mediums verschiedene Schichtdicken in der oberen Bestrahlungskammer 8 eingestellt werden.

Der Zweikammer-Photoreaktor 1 weist senkrecht zur Durchströmungsrichtung 20 Quecksilberniederdruck-Quarzlampen (15 W, NN 15/44 Original Hanau Quarzlampen GmbH, Hanau) auf, die über die Bestrahlungskammern 8, 9 von 80 cm Länge in gleichen Abständen verteilt sind, wobei jeder Strahler in einem zugeordneten Reflektor und die Lampen-Reflektorkombinationen jeweils in geringstmöglichem Abstand voneinander angeordnet sind. Der gesamte auf die Oberfläche des Mediums gelangende UV-Strahlungsfluss beträgt (unter Berücksichtigung der Reflektionsverluste von höchstens 45% sowie der Randverluste) ca. 60 W mit einer mittleren Bestrahlungsstärke E = 25 mW/cm². Die nachfolgende Tabelle 1 zeigt einen Vergleich des Zweikammer-Photoreaktors 1 mit einem Einkammer-Photoreaktor gleicher Gesamtschichtdicke; in der Tabelle 1 sind Werte für den Durchfluss Q-40 (m³/h) für eine Mindestdosis von 40 mWs/cm² für verschiedene Transmissionsfaktoren T (1 cm) und verschiedene Schichtdicken der oberen Bestrahlungskammer 8 angegeben.

Tabelle 1
Vergleich des Zweikammer-Photoreaktors 1 mit einem Einkammer-Photoreaktor gleicher Gesamtschichtdicke
Durchfluss Q-40 für Mindestdosis 40 mWs/cm²

| T (1 cm) | | 0.9 | | 0.7 | | 0.6 | | 0.6 |
| Q-40 | | m³/h | | m³/h | | m³/h | | m³/h |
| d | cm | | cm | | cm | | cm | |
| Schicht oben | 4 | 14.7 | 2 | 4.94 | 2 | 3.89 | 1 | 3.24 |
| Schicht unten | 2 | 5.74 | 2 | 2.42 | 2 | 1.4 | 2 | 2.33 |
| Zweikammer-Photoreaktor | 6 | 20.44 | 4 | 7.36 | 4 | 5.29 | 3 | 5.57 |
| Einkammer-Photoreaktor | 6 | 17.22 | 4 | 4.84 | 4 | 2.8 | 3 | 3.5 |
| Steigerungsfaktor | | 1.18 | | 1.52 | | 1.89 | | 1.59 |

60 W UV-254 nm auf 30 · 80 = 2400 cm² Einstrahlungsfläche; mittlere Bestrahlungsstärke E = 25 mW/cm². Einfluss des Transmissionsfaktors T (1 cm) und der Schichtdicken 2 in den Bestrahlungskammern.

Nach der vorstehenden Tabelle 1 eignet sich der Zweikammer-Photoreaktor für den Bereich der Transmissionsfaktoren T (1 cm) von 0.95 bis 0.5; bei T (1 cm) ≥ 0.9 soll die Schichtdicke der oberen Schicht 4 cm und mehr, bei T (1 cm) ≤ 0.6 ca. 1 cm betragen.

Für die Bereiche niedrigerer Transmissionsfaktoren werden die Schichtdicken auch der unteren Bestrahlungskammer 9 niedriger gewählt, z.B. 1 cm bei T (1 cm) = 0.4. Man erhält dann bei T (1 cm) = 0.4 unter den übrigen Bedingungen der obigen Tabelle für je 1 cm untere und obere Schicht zusammen Q–40 = 3.02 m³/h und der Steigerungsfaktor ist dann 1.75. Bei einer Schichtdicke der unteren Bestrahlungskammer 9 von 1 cm ist der Zweikammer-Photoreaktor 1 an die bei der Entkeimung von Abwasser vorkommenden Transmissionsbereiche optimal anpassungsfähig. Die durch die Verwendung von Reflektoren bedingten Verluste an wirksamer UV-Strahlung werden dabei durch die Unterteilung des Photoreaktors mehr als ausgeglichen, wodurch ein im Vergleich zum Einkammer-Photoreaktor energetisch günstigeres Ergebnis für die Entkeimung erzielt wird. Zweikammer-Photoreaktoren dieses Typs werden auch für die Entkeimung von Seewasser eingesetzt.

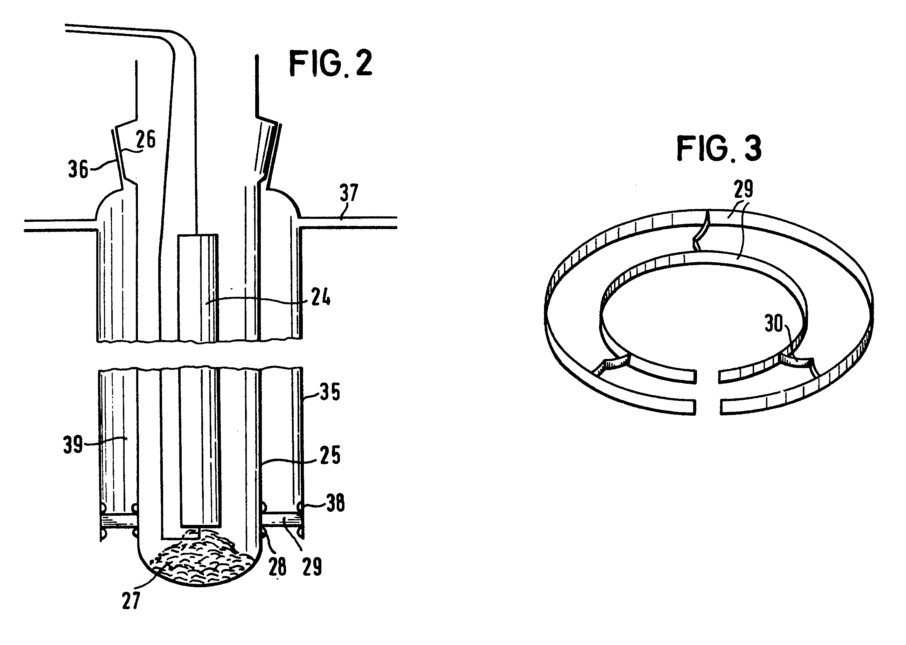

Fig. 2 bis 8 zeigen die Ausführung eines Mehrkammer-Photoreaktors, bei dem die Strahlungsquelle nach Art einer Tauchlampe ausgebildet ist.

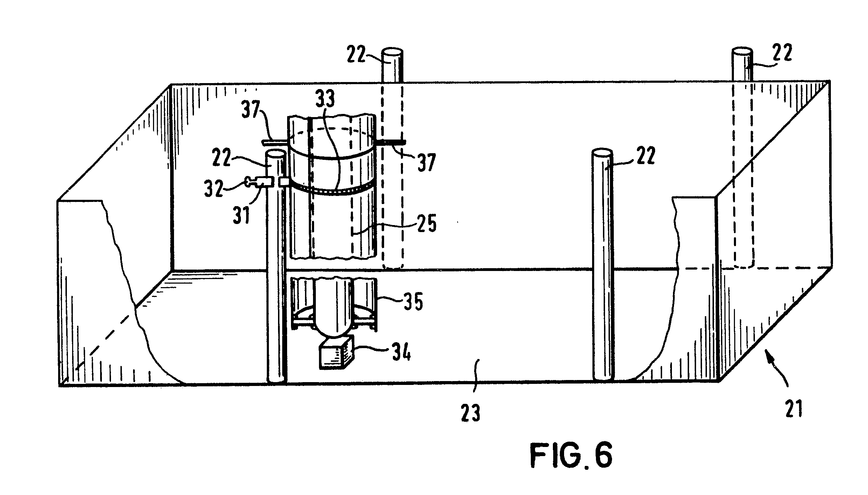

Fig. 2 zeigt im Längsschnitt eine erste Ausführung einer Teilanordnung des Mehrkammer-Photoreaktors mit einem Strahler 24 in einem Hüllrohr 25 aus Quarzglas, das in ein ebenfalls aus Quarzglas bestehendes Trennrohr 35 eingesetzt ist. Der Strahler 24 ist für Zwecke der Entkeimung eine antimondotierte Xenon-Hochdrucklampe; alternativ eignen sich dafür auch Quecksilberniederdruck-Quarzlampen bekannter Bauart. Für die Reinigung in Anwesenheit oder Abwesenheit von Oxidationsmitteln kann man auch Quecksilberhochdrucklampen oder andere Strahler geeigneter Emissionsbereiche verwenden. Der Strahler 24 ruht auf einer Auflage 27 am unteren Ende des Hüllrohres 25, die beispielsweise aus Glaswolle bestehen kann. An ihren oberen Enden sind das Hüllrohr 25 und das Trennrohr 35 über Schliffe 26, 36 miteinander verbunden, die durch geeignete, bekannte Sicherungen (Fa. Schott & Gen., Mainz) in dichtem Eingriff gehalten sind. Das Trennrohr 35 trägt nahe seinem oberen Ende zwei diametral gegenüberliegende Anschlüsse 37. Am unteren Ende der Teilanordnung ist eine Abstandshalterung vorgesehen, durch die das Hüllrohr 25 und das Trennrohr 35 über ihre Länge in gleichem Abstand zueinander gehalten werden. Die Abstandshalterung besteht aus zwei konzentrisch zueinander angeordneten Federringen 29, die durch drei im Winkel von 120° gegeneinander versetzte Stege 30 aus federndem Material verbunden sind (siehe Fig. 3). Die Federringe 29 sind zwischen kleinen Vorsprüngen 28, 38 gehalten, die in Winkelabständen von ca. 120° und in einem dem entsprechenden Mass des Federringes 29 angepassten Axialabstand an der Aussenseite des Hüllrohres 25 bzw. an der Innenseite des Trennrohres 35 angeordnet sind. Diese Abstandshalterung kann zur Einstellung eines über die Durchtrittsfläche gleichförmigen Strömungsprofils noch mit einer Lochplatte 5 versehen werden, wie im Zusammenhang mit Fig. 5 erläutert wird.

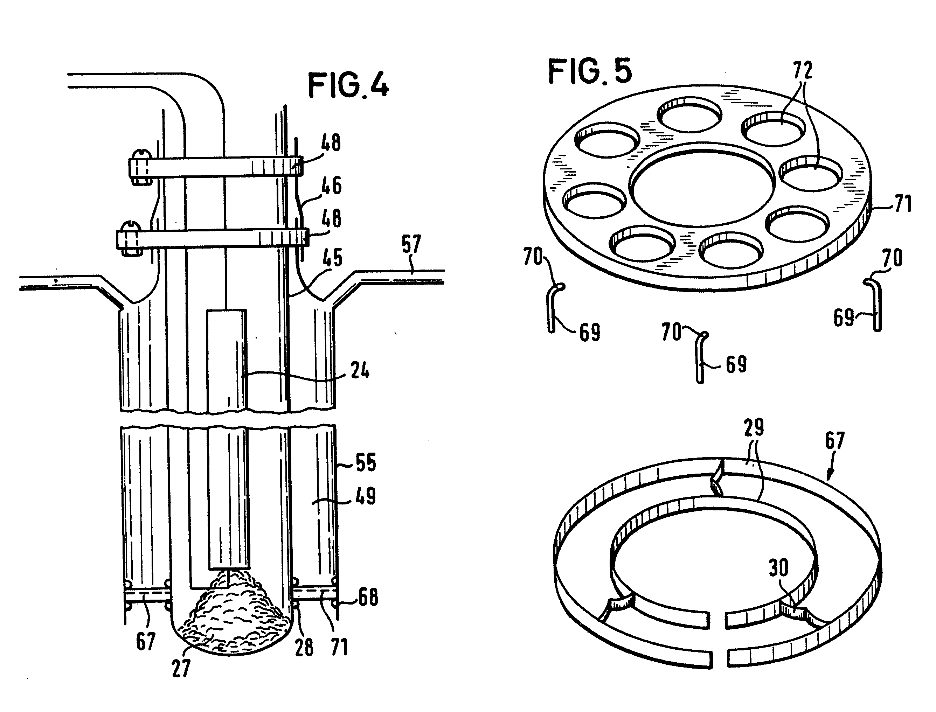

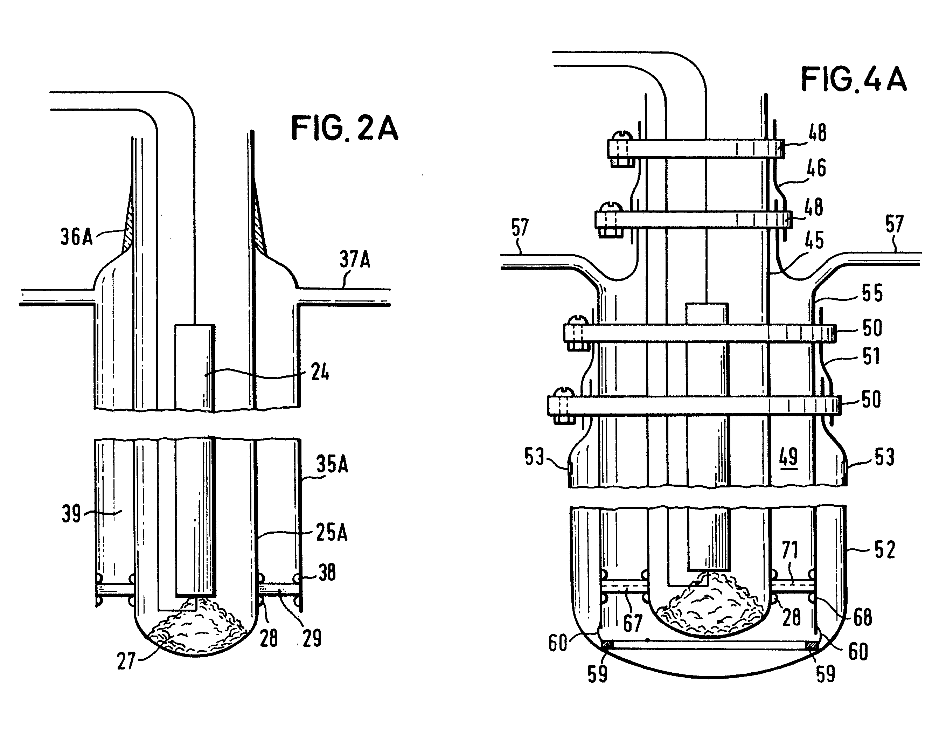

Fig. 4 zeigt eine abgewandelte Teilanordnung ähnlich Fig. 2. In einem Hüllrohr 45 aus Quarzglas befindet sich ein Strahler 24 der vorgenannten Art. Es ist von einem Trennrohr 55 aus Quarzglas umgeben, das an seinem oberen Ende eine Verengung, die geringfügig weiter ist als das Hüllrohr 45, und nahe seinem oberen Ende zwei diametral gegenüberliegende Anschlüsse 57 trägt. Das Hüllrohr 45 und das Trennrohr 55 sind konzentrisch zueinander angeordnet und an ihren oberen Enden durch eine übergreifende Dichtmanschette 46 aus einem elastischen Kunststoff, der gegen die UV-Strahlung und das durchströmende Medium beständig ist, miteinander abdichtend verbunden. Die Dichtmanschette 46 ist durch Ligaturen 48, die nach Art von Schlauchschellen ausgebildet sind, gesichert. Am unteren Ende der Teilanordnung ist eine Abstandshalterung 67 vorgesehen, durch die das Hüllrohr 45 und das Trennrohr 55 über ihre Länge in gleichem Abstand zueinander gehalten werden. Die Abstandshalterung 67 (Fig. 5) ist entsprechend Fig. 2 zwischen Vorsprüngen 28 am Hüllrohr 45 und Vorsprüngen 68 am Trennrohr 55 angeordnet und besteht zunächst aus einem doppelten Federring 29 mit federnden Stegen 30. Der äussere Federring 29 ist mit axial von seinem Umfang nach oben vorstehenden Trägern 69 versehen, deren Enden 70 radial nach innen umgebogen sind und dadurch eine Platte 71, die Durchtrittsöffnungen 72 besitzt, in Anlage an dem Federring 29 halten. Die Durchtrittsöffnungen 72 in der Platte 71 sind der von dem Hüllrohr 45 und dem Trennrohr 55 gebildeten Bestrahlungskammer 49 zugeordnet und gleichmässig über die jeweilige Durchtrittsfläche verteilt. Die Platte 71 besteht aus gegen UV-Strahlung und gegen das durchströmende Medium beständigem Material, das keine Verunreinigungen an das durchströmende Medium abgibt (rostfreier Stahl, beschichtete Metalle, Kunststoff, Keramik, Glas, Quarz). Die Weite der Durchtrittsöffnungen 72, die kreisförmigen oder anderen Querschnitt haben können, ist so gross, dass sie die Strömung nicht wesentlich behindern, aber doch ein über die Durchtrittsfläche gleichförmiges Strömungsprofil erzeugen. Die ganze Anordnung ist so getroffen, dass sich die Träger 69 jeweils zwischen den Vorsprüngen 68 an der Innenwand des Trennrohres 55 befinden.

Gegenüber den vorstehend beschriebenen Ausführungen sind eine Reihe von Abwandlungen möglich. In der in Fig. 2A dargestellten Ausführung ist das offene Ende des Trennrohres 35A mit dem Hüllrohr 25A unter Ausbildung eines einheitlichen Bauteils verschmolzen, das aber aufwendig in der Herstellung und empfindlich in der Handhabung ist. In einer einfacheren Ausführung kann die Abstandshalterung auch allein von der mit Durchtrittsöffnungen 72 versehenen Platte 71 gebildet werden; dabei fällt dann der obere Kranz der Vorsprünge 28 und 68 fort, und die Platte 71 wird durch einen Sprengring in Auflage an dem unteren Kranz der Vorsprünge 28 und 68 gehalten.

In einer Abänderung der Teilanordnung von Fig. 4 ist ein zusätzliches Quarzglasrohr 52 (siehe Fig. 4A) koaxial zu den Quarzglasrohren 45 und 55 angeordnet. Das Quarzglasrohr 52 ist am unteren Ende geschlossen; das obere offene Ende verjüngt sich und ist durch eine mit Ligaturen 50 gesicherte, überlappende Manschette 51 abdichtend mit dem Quarzglasrohr 55 in ähnlicher Weise verbunden wie die Quarzglasrohre 45 und 55. Das verjüngte Ende ist dicht unterhalb der Anschlüsse 57 an dem Trennrohr 55 befestigt. Mindestens zwei Durchtrittsöffnungen 53 in der Wandung des Quarzglasrohres 52 nahe seinem verjüngten Ende sind gleichmässig über dessen Umfang verteilt. Das Trennrohr 55 erstreckt sich bis zu einer dem Boden des Quarzglasrohres 52 innen aufliegenden Halterung aus einem Ring 59, von dem eine Anzahl von das Ende des Trennrohres 55 aufnehmenden und halternden Blattfedern 60 vorstehen. Zwischen dem Rand des Trennrohres 55 und dem Ring 59, sowie den Blattfedern 60 besteht ausreichender Zwischenraum für den ungehinderen Durchfluss des Mediums zwischen den durch das Trennrohr 55 getrennten Bestrahlungskammern. Es kann aber auch der Rand des Trennrohres 55 mit Ausschnitten für die Verbindung zwischen den Bestrahlungskammern versehen sein und dann dem Ring 59 direkt aufliegen. Der Ring 59 und die Blattfedern 60 bestehen aus Material, das gegen UV-Strahlung und gegen das durchströmende Medium beständig ist und selbst keine störenden Verunreinigungen an das durchströmende Medium abgibt (rostfreier Stahl, beschichtete Metalle, vorzugsweise mit fluorierten Kohlenwasserstoffpolymeren, Kunststoffe, Keramik, Quarz, Glas).

Die in Fig. 2, 2A, 4 und 4A dargestellten Teilanordnungen für den Strahler 24 bilden gemeinsam mit einem Tank 21 den Durchflussreaktor 41 des Zweikammer-Photoreaktors 20. Gemäss Fig. 6 trägt der Tank 21 an seinen Längswänden Träger 22, an denen jeweils eine der in Fig. 2 abgebildeten Teilanordnungen befestigt ist. Der Tank 21 und die Träger 22 bestehen aus rostfreiem Stahl und sind aneinandergeschweisst. Der Tank 21 und die Träger 22 können aber auch aus verschiedenen Materialien bestehen, die in geeigneter Weise fest miteinander verbunden sind; dabei ist der Tank 21 aus einem Material gefertigt, das UVbeständig ist und allen sonstigen Anforderungen, z.B. lebensmittelrechtlichen Bestimmungen, genügt. In den oben offenen Tank 21 mündet der (nicht dargestellte) Abfluss einer Quelle für das zu bestrahlende Medium; der Tank 21 kann aber gegebenenfalls auch über einen Anschlusstutzen und eine Verbindungsleitung an die Quelle des zu bestrahlenden Mediums angeschlossen sein.

In der Kombination der Teilanordnung nach Fig. 4A mit dem Tank 21 besteht der Abfluss zweckmässigerweise aus einem vom Boden des Tanks 21 ausgehenden Überlaufrohr, das sich bis zur Höhe der Durchtrittsöffnungen 53 erstreckt. Dadurch wird die erwünschte konstante Füllhöhe des Tanks 21 sichergestellt.

Die Teilanordnung nach einer der Fig. 2 bis 4 wird mit geeigneten Mitteln an dem Träger 22 gehaltert; dazu ist eine Muffe 31 mit einer Feststellschraube 32 vorgesehen, die eine gegebenenfalls mit einem Schutzüberzug überzogene Kette 33 trägt, die die Teilanordnung umgibt und entsprechend deren Umfang an der Muffe 31 eingehängt ist. Solche Halterungen im Zusammenhang mit Bestrahlungsgeräten sind bekannt und im Handel erhältlich, so das sie hier nicht im einzelnen beschrieben werden müssen. Die Teilanordnung ist in Fig. 6 nur schematisch dargestellt. Am Grunde des Tanks 21 befinden sich Auflagen 34, denen die Teilanordnung aufsitzt, wodurch zusätzlich Sicherheit der Halterung erreicht wird.

Für den Betrieb des Durchflussreaktors 41 werden die Anschlüsse 37 bzw. 57 der an den Trägern 22 gehaltenen Teilanordnungen nach einer der Fig. 2 bis 4 zu einer gemeinsamen (nicht gezeigten) Ableitung miteinander verbunden. Das eintretende Medium durchsetzt zunächst den die erste Bestrahlungskammer 23 bildenden Tank 21; es tritt dann durch die von dem Hüllrohr 25 bzw. 45 und dem Trennrohr 35 bzw. 55 gebildete innere Bestrahlungskammer 39 bzw. 49 und deren Anschluss 37 bzw. 57 hindurch in die (nicht dargestellte) Ableitung aus.

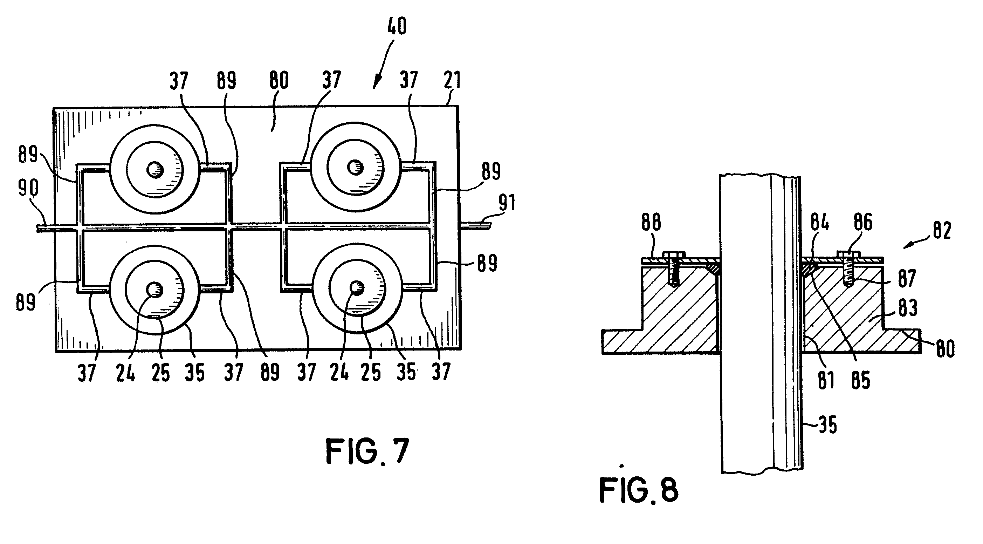

Fig. 7 und 8 zeigen eine weitere Ausführungsform des Durchflussreaktors 41 für einen Zweikammer-Photoreaktor 40 entsprechend Fig. 6, bei dem der Tank 21, der einen Anschlusstutzen 91 trägt, durch einen Deckel 80 verschlossen ist, der mit Durchführungen 81 und Halterungen 82 versehen ist, an denen die in Fig. 2 gezeigte Teilanordnung abgedichtet gehaltert ist. Die Halterungen 82 bestehen jede aus einem von dem Deckel 80 hochstehenden Kragen 83, in dem das jeweils äussere Quarzglasrohr 35 geführt ist. Das Quarzglasrohr 35 trägt einen O-Ring 84, der einer Schrägfläche 85 an der oberen Innenkante des Kragens 83 anliegt und durch einen mit Schrauben 86, die in Gewindebohrungen 87 an der Oberseite des Kragens 83 eingreifen, gehaltenen Anpressring 88 gesichert ist. Die Anschlüsse 37 der Teilanordnungen nach Fig. 2 werden über Verbindungsleitungen 89 an eine gemeinsame Ableitung 90 angeschlossen. In gleicher Weise können die Teilanordnungen nach Fig. 4 oder die übrigen vorstehend beschriebenen Teilanordnugnen an dem Deckel 80 abdichtend gehaltert werden.

Die offene Anordnung des Mehrkammer-Photoreaktors 20 wird mit Vorteil beispielsweise bei Klimawäschern verwendet, deren Auslauf sich direkt oberhalb des Tanks 21 befindet; die geschlossene Anordnung des Mehrkammer-Photoreaktors 40 ermöglicht andere Anwendungen, bei denen das Medium ohne Überdruck im Umlaufverfahren bestrahlt werden soll. Um eine Unterschreitung der geforderten Mindestdosis sicher zu vermeiden, wird auch hier in die Zuleitung zweckmässig ein Durchflussbegrenzer der weiter oben beschriebenen Art eingebaut. Bei den Mehrkammer-Photoreaktoren 20, 40 wird der Nachteil der Inhomogenität der Bestrahlungsstärkeverteilung in der von dem Tank 21 gebildeten ersten Bestrahlungskammer 23 dadurch kompensiert, dass das Medium durch die innere Bestrahlungskammer 39 bzw. 49 hindurchgeleitet wird, in der es unter definierten Bedingungen mit einem geringeren Gradienten der Bestrahlungsstärke einer hohen Mindestbestrahlungsstärke ausgesetzt wird. Je nach den Anforderungen kann dabei eine kleinere oder grössere Anzahl der Teilanordnungen nach Fig. 2 bzw. 4 in den Mehrkammer-Photoreaktor 20, 40 eingesetzt werden. Für die Funktion des Mehrkammer-Photoreaktors 20, 40 kommt es nicht entscheidend auf die Durchflussrichtung an. Will man mit Sicherheit hohe Entkeimungsgrade erzielen, so dürfte es zweckmässig sein, das Medium durch die innere Bestrahlungskammer 39 bzw. 49 zuletzt zu leiten. Will man jedoch während der Bestrahlung eine Begasung des Mediums mit z.B. Sauerstoff vornehmen, so empfiehlt sich die umgekehrte Durchflussrichtung.

Für die Reinigung, insbesondere für die Entkeimung oder Desinfektion von Medien, die mit hoher Leistung durch einen Durchflussreaktor mit einer überwiegend im Bereich zwischen 240 und 320 nm emittierenden UV-Strahlungsquelle gefördert werden sollen, eignen sich besonders solche Vorrichtungen, bei denen der Durchflussreaktor und die Strahlungsquelle ringförmig zueinander angeordnet sind. Dabei kann ein ringförmiger Durchflussreaktor eine im Inneren angeordnete Strahlungsquelle umgeben; es kann aber auch eine äussere Strahlungsquelle in Form einer Reihe von Strahlern in jeweils zugeordneten Reflektoren, die den Durchflussreaktor kranzförmig umgeben, oder auch beide Arten von Strahlungsquellen vorgesehen sein. Der Durchflussreaktor kann auch rohrförmig ausgebildet sein und ist dann mit einer äusseren Strahlungsquelle kombiniert. Die folgende Tabelle 2 zeigt für einen ringförmigen Durchflussreaktor mit einem Innendurchmesser $D_i = 4$ cm und einem Aussendurchmesser $D_a = 6$ bis 14 cm den Abfall der inneren Bestrahlungsstärke E bei radialer Durchstrahlung eines Mediums mit einem Transmissionsfaktor T (1 cm) = 0.6. Befindet sich im Inneren dieses Durchflussreaktors in axialer Position eine Quecksilberniederdruck-Quarzlampe von 1 m effektiver Länge, so strahlt deren radiale Ausstrahlung auf dieser Länge 15 Watt UV-254 nm an der durchstrahlten Innenfläche des Durchflussreaktors in das Medium ein.

Bei einer effektiven Einstrahlungsfläche von $\pi \cdot D_i \cdot 100$ cm² = 1256.6 cm² ist die mittlere Bestrahlungsstärke in dieser Fläche $E_i$ = 11.94 mW/cm². Die Spalten der Tabelle 2 zeigen die Durchmesser D und Schichtdicken d, hierzu die Geometriefaktoren G und die Transmissionen T sowie als deren Produkt $G \cdot T = E_{rel}$ die relative Bestrahlungsstärke in den Schichten; Spalte $E_d$ zeigt die innere Bestrahlungsstärke in den Schichten; die Spalte $V_d$ zeigt die zugehörigen Ringkammervolumina. Die letzte Spalte der Tabelle 2 zeigt die zugehörigen Durchflüsse Q–40 in m³/h bei Einhaltung einer Mindestbestrahlungsdosis von 40 mWs/cm², berechnet für eine gleichförmige Strömung.

Tabelle 2
Bestrahlungsstärken $E_d$ und Durchflüsse Q-40 von ringförmigen
Einkammer-Photoreaktoren verschiedener Schichtdicken d

| D cm | d cm | G | T | $E_{rel(d)}$ G·T | $E_d$ mW/cm² | $V_d$ ml | Q-40 m³/h |
|---|---|---|---|---|---|---|---|
| 4 | 0 | 1.0 | 1.0 | 1.0 | 11.94 | 0 | 0 |
| 6 | 1 | 0.666 | 0.6 | 0.399 | 4.76 | 1571 | 0.67 |
| 8 | 2 | 0.5 | 0.36 | 0.18 | 2.15 | 3770 | 0.73 |
| 10 | 3 | 0.4 | 0.216 | 0.086 | 1.03 | 6597 | 0.61 |
| 12 | 4 | 0.333 | 0.126 | 0.042 | 0.53 | 10053 | 0.47 |
| 14 | 5 | 0.286 | 0.078 | 0.022 | 0.26 | 14137 | 0.33 |

15 W UV-254 nm auf 1 m Länge in axialer Position;
Hüllrohr $D_i$ = 4 cm

Man erkennt aus der Tabelle 2, dass die innere Bestrahlungsstärke $E_d$ mit zunehmender Schichtdicke d stark abnimmt, während im Gegensatz dazu das Ringkammervolumen $V_d$ beträchtlich zunimmt. Bei einem Transmissionsfaktor des zu bestrahlenden Mediums von T (1 cm) = 0.6 wird Q-40 = 0.73 m³/h als Maximum bei einer Schichtdicke von d = 2 cm gefunden (siehe Tabelle 2). Bei grösseren Schichtdicken d nimmt Q-40 ab, weil der Einfluss der grossen Ringkammervolumina, die nur einer relativ geringen inneren Bestrahlungsstärke $E_d$ ausgesetzt sind, überwiegt. Für Medien mit anderen Transmissionsfaktoren liegen die erzielbaren Durchflussmaxima für Q-40 bei anderen Schichtdicken d: Bei T (1 cm) = 0.7 ist Q-40 (max) = 1.32 m³/h bei 2 cm Schichtdicke; bei T (1 cm) = 0.8 erreicht Q-40 einen Maximalwert von 1.95 m³/h bei d = 4 cm, bei T (1 cm) = 0.9 wird bei einer Schichtdicke von 5 cm Q-40 = 3.43 m³/h. Bei konstanter Schichtdicke d = 5 cm des Einkammer-Photoreaktors werden bei T (1 cm) = 0.9; 0.8; 0.7 Durchflüsse Q-40 = 2.56; 1,42; 0.73 m³/h erhalten.

Die folgende Tabelle 3 zeigt die Verhältnisse für ein Medium ebenfalls mit T (1 cm) = 0.6, jedoch bei einem Mehrkammer-Photoreaktor mit gleichen Abmessungen, der durch UV-durchlässige Trennwände (mit vernachlässigten Dimensionen) in Bestrahlungskammern von jeweils 1 cm Schichtdicke unterteilt ist. Die ersten 6 Spalten der Tabelle 3 enthalten die gleichen Angaben wie Tabelle 2. In die Spalten $V_k$ sind die Volumina der einzelnen Bestrahlungskammern eingetragen und in die Spalte Q-40 (k) die Durchflüsse, bei denen in jeder einzelnen Bestrahlungskammer die Mindestbestrahlungsdosis von 40 mWs/cm² einwirkt. Die letzte Spalte der Tabelle 3 zeigt die Bestrahlungsdosen E · t (k) in mWs/cm², die das alle Bestrahlungskammern nacheinander durchfliessende Medium bei einem Durchfluss von 1.61 m³/h in jeder einzelnen Bestrahlungskammer erhält.

Tabelle 3
Bestrahlungsstärken $E_d$ und Durchflüsse Q-40 eines in 5 Schichten von je
1 cm Schichtdicke unterteilten ringförmigen Mehrkammer-Photoreaktors

| D cm | d cm | G | T | $E_{rel}$ G·T | $E_d$ mW/cm² | $V_k$ ml | Q-40(k) m³/h | E·t(k) bei Q = 1.61 m³/h mWs/cm² |
|---|---|---|---|---|---|---|---|---|
| 4 | 0 | 1.0 | 1.0 | 1.0 | 11.94 | 0 | 0 | – |
| 6 | 1 | 0.666 | 0.6 | 0.399 | 4.76 | 1571 | 0.67 | 16.72 |
| 8 | 2 | 0.5 | 0.36 | 0.18 | 2.15 | 2199 | 0.425 | 10.57 |
| 10 | 3 | 0.4 | 0.216 | 0.086 | 1.03 | 2827 | 0.26 | 6.51 |
| 12 | 4 | 0.333 | 0.126 | 0.042 | 0.53 | 3456 | 0.16 | 4.09 |
| 14 | 5 | 0.286 | 0.078 | 0.022 | 0.26 | 4084 | 0.097 | 2.37 |
| | | | | | | | 1.61 | 40.27 |

15 W UV-254 nm auf 1 m Länge in axialer Position;
Hüllrohr $D_i$ = 4 cm.

Aus der vorletzten Spalte der Tabelle 3 ergibt sich, dass bei parallelem Durchfluss durch die Bestrahlungskammern in der Weise, dass in jeder Schicht die Mindestbestrahlungsdosis von 40 mWs/cm² eingehalten wird, ein Gesamtdurchfluss von Q-40 (gesamt) = 1.61 m³/h möglich ist. Entsprechend ist aus der letzten Spalte der Tabelle 3 zu entnehmen, dass bei Hintereinanderschaltung der Bestrahlungskammern und bei einem Druckfluss von 1.61 m³/h das Medium mit einer Gesamtdosis von ca. 40 mWs/cm² bestrahlt worden ist. Für ein Medium mit einem Transmissionsfaktor T (1 cm) = 0.7 ist Q-40 (gesamt) = 2.28 m³/h, für T (1 cm) = 0.8 ist der entsprechende Wert Q-40 (gesamt) = 3.15 m³/h, für T (1 cm) = 0.9 ist Q-40 (gesamt) = 4.37 m³/h.

Insgesamt zeigt die vorstehende Diskussion in Verbindung mit den Tabellen 2 und 3, dass die Unterteilung des Photoreaktors eine erhebliche Leistungssteigerung erbringt. Dieses Ergebnis ist in Tabelle 4 zusammengestellt. Die Zeilen der Tabelle 4 enthalten für Transmissionsfaktoren T (1 cm) = 0.6 bis 0.9 die Durchflüsse Q-40 (max) bei den jeweils optimalen Schichtdicken, die Durchflüsse Q-40 des Einkammer-Photoreaktors entsprechend Tabelle 2 bei einer Schichtdicke von d = 5 cm und die Durchflüsse Q-40 (gesamt) bei einem Mehrkammer-Photoreaktor mit 5 Bestrahlungskammern von je 1 cm Schichtdicke (Gesamtschichtdicke d = 5 cm), sowie die Steigerungsfaktoren F der Durchflüsse Q-40 des Mehrkammer-Photoreaktors gegenüber dem Einkammer-Photoreaktor.

Tabelle 4
Leistungsvergleich von Ein- und Mehrkammer-Photoreaktoren für Medien verschiedener Transmissionsfaktoren

| | T (1 cm) | 0.9 | 0.8 | 0.7 | 0.6 |
|---|---|---|---|---|---|
| Q-40 (max) | m³/h | 2.56 | 1.46 | 0.99 | 0.73 |
| Q-40 | m³/h | 2.56 | 1.42 | 0.73 | 0.33 |
| Q-40 (gesamt) | m³/h | 4.37 | 3.15 | 2.28 | 1.61 |
| F | | 1.70 | 2.16 | 3.13 | 4.88 |

Aus Tabelle 4 sind die Vorteile des Mehrkammer-Photoreaktors gegenüber den bekannten Einkammer-Photoreaktoren unmittelbar ersichtlich. Allein durch die beschriebene Unterteilung der Bestrahlungskammer des Einkammer-Photoreaktors zum Mehrkammer-Photoreaktor lassen sich ohne zusätzliche Strahlungsquellen oder sonstige Massnahmen Leistungssteigerungen von über 10% erzielen. Das bedeutet, dass bei gleicher Bestrahlungsdosis der Durchfluss bzw. bei gleichem Durchfluss die applizierte Bestrahlungsdosis verdoppelt werden kann. Derartige Effekte lassen sich durch keine irgendwie geartete Kombination von Einkammer-Photoreaktoren erreichen. Die erzielten Leistungssteigerungen sind sowohl bei Parallelschaltung der Bestrahlungskammern als auch bei Serienschaltung der Bestrahlungskammern in den Beispielen gleich. In der Praxis bringt jedoch die Serienschaltung erhebliche zusätzliche Vorteile. So bietet die Serienschaltung der Bestrahlungskammern eine wesentlich erhöhte Sicherheit gegenüber Strömungskurzschlüssen und zusätzlich eine wesentlich verbesserte Durchmischung des zu bestrahlenden Mediums im gesamten Strahlungsfeld. Durch die Serienschaltung von Bestrahlungskammern wird nämlich das strömende Medium in wechselnden Richtungen durch die Bestrahlungszone geführt, wobei durch die erzwungene Umsteuerung der strömenden Schichten eine Neuorientierung der Flüssigkeitspartikeln auf dem Weg durch die Bestrahlungskammern erfolgt. Da bei der Serienschaltung zudem mit relativ höheren Strömungsgeschwindigkeiten, besonders in den inneren Bestrahlungskammern, gearbeitet wird, sind Strömungsverhältnisse mit wesentlich höheren Reynolds-Zahlen gegenüber den Einkammer-Photoreaktoren möglich. Dies hat zusätzlich zur besseren Durchmischung einen günstigen Effekt bei der Unterdrückung von Niederschlagsbildungen.

Tabelle 4 zeigt insbesondere auch, dass die Steigerungsfaktoren F bei abnehmenden Transmissionsfaktoren, aber konstanter Schichtdicke stark zunehmen. Dies ergibt sich daraus, dass der Einkammer-Photoreaktor für jeden Transmissionsfaktor eine optimale Schichtdicke besitzt, d.h. solche Photoreaktoren sind nur wenig anpassungsfähig an Medien mit veränderlichen bzw. unterschiedlichen Transmissionsfaktoren. Demgegenüber besitzt ein Mehrkammer-Photoreaktor den grossen Vorteil, dass er auch bei Medien mit stark veränderlichen bzw. unterschiedlichen Transmissionsfaktoren günstige Leistungen erbringt. Das Bestrahlungsergebnis beim Mehrkammer-Photoreaktor wird also bei Medien mit niedrigem Transmissionsfaktor nicht dadurch beeinträchtigt, dass erhebliche Anteile der Gesamtschicht nur minimale Bestrahlungsdosen erhalten, und andererseits erlaubt der Mehrkammer-Photoreaktor bei hohem Transmissionsfaktor des Mediums die Ausnutzung des gegebenen Strahlungsflusses durch die hohe Gesamtschichtdicke aller Bestrahlungskammern.

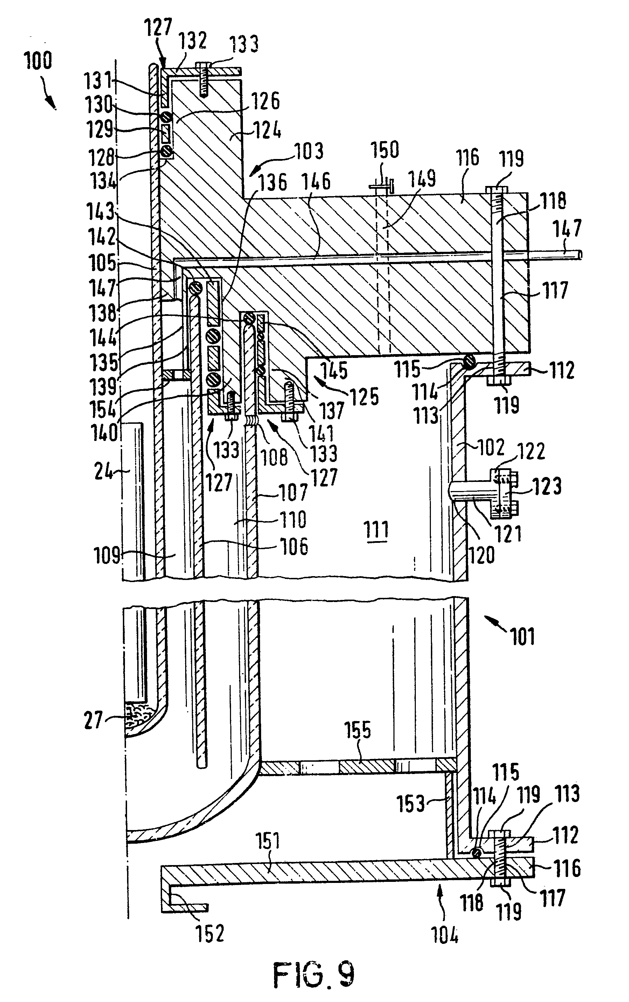

Mehrkammer-Photoreaktoren mit ringförmiger Anordnung von Strahlungsquelle und Durchflussreaktor sind aus mehreren Rohrstücken aus Quarzglas aufgebaut, die ineinander angeordnet sind und deren Durchmesser so gewählt sind, dass koaxiale Bestrahlungskammern der gewünschten Schichtdicke gebildet werden. Solche Quarzglasrohre können mit der gewünschtern Genauigkeit der Abmessungen hergestellt werden und sind mit geeigneten Durchmessern und Wandstärken im Handel erhältlich. Die Quarzglasrohre werden in bekannter Weise zueinander zentriert und zwischen Verschlussteilen (siehe weiter unten) gehaltert, die den Durchflussreaktor stirnseitig abschliessen. Die Verschlussteile besitzen z.B. durch Stopfbuchspakkungen abgedichtete Halterungsnuten für die Quarzglasrohre und sind mit Innenkanälen und Anschlusstutzen versehen, durch die die Zuleitung und Ableitung des Mediums bei Parallelschaltung und bei Serienschaltung der Bestrahlungskammern deren Verbindung untereinander bewirkt wird. In den Abbildungen 9 bis 12 sind Ausführungsbeispiele ringförmiger Mehrkammer-Photoreaktoren mit Innenbestrahlung, mit einer Druckausgleichseinrichtung und mit Aussenbestrahlung dargestellt.

Ein für Innenbestrahlung eingerichteter Dreikammer-Photoreaktor 100 ist in Abbildung 9 zur Hälfte im Längsschnitt dargestellt. Er enthält einen Strahler 24 der vorgenannten Art, der zur Erhöhung der Bestrahlungsstärke im Photoreaktor 100 einfach oder mehrfach gewendelt sein kann. Der Strahler 24 ist achsnah im Inneren eines Durchflussreaktors 101 angeordnet, der von einem strahlungsundurchlässigen Aussenmantel 102, von einem ersten Verschlussteil 103 und einem zweiten Verschlussteil 104 und von einem strahlungsdurchlässigen inneren Hüllrohr 105, das in dem ersten Verschlussteil 103 gehaltert ist, gebildet ist. Das innere Hüllrohr 105 ist ein einseitig geschlossenes Quarzglasrohr, an dessen geschlossenem Ende der Strahler 24 auf einer Glaswollepackung 27 aufliegt. Der Durchflussreaktor 101 ist durch ein Quarzglasrohr 106 und ein einseitig geschlossenes Quarzglasrohr 107 mit Durchtrittsöffnungen 108 in der Wand an seinem offenen Ende, die beide ebenfalls in dem ersten Verschlussteil 103 gehalten sind, in drei Bestrahlungskammern 109, 110, 111 unterteilt.

Der Aussenmantel 102 ist zur Verbindung mit den Verschlussteilen 103, 104 an beiden Enden mit Ringflanschen 112 versehen, die längs ihres Umfangs verteilte Bohrungen 113 aufweisen. An der Aussenseite der Ringflansche 112 befinden sich Ausnehmungen 114 zur Aufnahme von abdichtenden O-Ringen 115. Die Verschlussteile 103 und 104 tragen Flansche 116 mit längs ihres Umfangs verteilten Bohrungen 117, deren Zahl und Duchmesser den Bohrungen 113 in den Ringflanschen 112 des Aussenmantels 102 entsprechen. Der Aussenmantel 102 und die Verschlussteile 103, 104 werden mit den Ringflanschen 112 und den Flanschen 116 so angeordnet, dass die Bohrungen 113 und 117 fluchten, so dass diese Teile durch Gewindebolzen 118, die sich durch die Bohrungen 113 und 117 erstrecken, und Muttern 119 fest miteinander verbunden werden können.

Der Aussenmantel 102 ist zu Beobachtungs- oder Kontrollzwecken im Bereich des Strahlungsfeldes des Strahlers 24 mit einer Öffnung 120 versehen, in die ein Tubus 121 mit einem äusseren Ringflansch 122 eingepasst ist. Bei Nichtgebrauch ist der Tubus 121 durch einen fest und dicht, z.B. durch Verschrauben, mit dem Ringflansch 122 verbundenen Deckel 123 verschlossen. Im Gebrauch ist der Tubus 121 über ein Quarzfenster mit dem Photodetektor einer Überwachungseinrichtung für die durch den Durchflussreaktor 101 hindurchtretende Strahlung verbunden. Der Aussenmantel 102 kann zwecks Ausnutzung der bei hohem Transmissionsfaktor des Mediums auf den Aussenmantel 102 aufgestrahlten UV-Leistung mit einem die UV-Strahlen in das Medium reflektierenden Material versehen werden. Bei Verwendung eines Aussenmantels aus Quarz lässt sich die reflektierende Oberfläche auch auf der Aussensetie anordnen, wodurch Beeinflussungen des Reflektionsvermögens durch das Medium vermieden werden.

Der Aussenmantel 102 und die Verschlussteile 103, 104 bestehen aus Metall wie rostfreiem Stahl, aus Metallen mit einem Schutzüberzug aus Glas, Emaille oder Kunststoff, aus verzinktem Eisenblech, aus Keramik; es kann dafür jedes Material geeigneter mechanischer Festigkeit Verwendung finden, das beständig gegen UV-Strahlung ist und keine Fremdstoffe oder Schadstoffe an das durchfliessende Medium abgibt. Zur Erhöhung der mechanischen Festigkeit und zur Erleichterung der Verarbeitung und Handhabung können das Hüllrohr 105 und die Quarzglasrohre 106, 107 in den Bereichen, die ausserhalb des Strahlungsfeldes des Strahlers 24 liegen, mit Verlängerungsstücken, z.B. aus Sinterquarz, verschmolzen sein.

Das Verschlussteil 103 ist allgemein ringförmig ausgebildet und hat einen Innendurchmesser, der eng an den Aussendurchmesser des Hüllrohres 105 angepasst ist. Das ringförmige Verschlussteil 103 trägt zwei Axialteile 124, 125, die sich zu beiden Seiten des Flansches 116 an dessen Innenrand erstrecken und zur Halterung des Hüllrohres 105 bzw. der Quarzglasrohre 106 und 107 dienen. Das erste Axialteil 124 ist an seinem Aussenende mit einer Gegenbohrung 126 versehen, in die eine Stopfbuchspackung 127 eingesetzt ist. Die Stopfbuchspackung 127 besteht aus zwei durch einen Führungsring 129 getrennten O-Ringen 128, 130, die durch einen Anpressring 131 mit einem Ringflansch 132, der durch Schrauben 133 an der Aussenfläche des ersten Axialteils 124 befestigt ist, gegen die am Ende der Gegenbohrung 126 ausgebildete Schulter 134 gedrückt werden. Dadurch wird das Hüllrohr 105 fest und abgedichtet an dem ersten Axialteil 124 gehalten. Das zweite Axialteil 125 ist von innen her mit drei konzentrischen Ringnuten 135, 136 und 137 versehen, deren Tiefe von innen nach aussen abnimmt und die ringförmige Stege 138, 139, 140 und 141 ausbilden. Die Stege 138 und 139 besitzen geringe und unterschiedliche axiale Tiefe und begrenzen die innerste, tiefste Ringnut 135. Die mittlere Ringnut 136 wird von dem Steg 139 und dem längeren Steg 140 begrenzt, während die äusserste, flachste Ringnut 137 von zwei gleich tiefen Stegen 140, 141 eingeschlossen ist. Die mittlere Ringnut 136 dient zur Aufnahme des Quarzglasrohres 106, dessen Ende über einen O-Ring 142 dem Boden der Ringnut 136 anliegt; eine Buchse 143 umschliesst den O-Ring 142 und das obere Ende des Quarzglasrohres 106. Das Quarzglasrohr 106 wird durch eine Stopfbuchspackung 127, die mit Schrauben 133 an der Aussenfläche des Stegs 140 befestigt ist, fest und abgedichtet in der mittleren Ringnut 136 gehalten. Die äussere Ringnut 137 dient zur Aufnahme des einseitig geschlossenen Quarzglasrohres 107, dessen offenes Ende über einen O-Ring 144 dem Boden der Ringnut 137 anliegt; eine Buchse 145 umschliesst den O-Ring 144 und das offene Ende des einseitig geschlossenen Quarzglasrohres 107. Das Quarzglasrohr 107 wird durch eine Stopfbuchspackung 127, die mit Schrauben 133 an der Aussenfläche des Stegs 141 befestigt ist, fest und abgedichtet oberhalb der Durchtrittsöffnungen 108 in der äusseren Ringnut 137 gehaltert.

Das Verschlussteil 103 besitzt zwei diametral gegenüber in der Umfangsfläche des Flansches 116 mündende Radialkanäle 146, die in Anschlussstutzen 147 enden. An ihrem inneren Ende sind die Radialkanäle 146 mit einem rechtwinklig abzweigenden Axialkanal 148 verbunden, der in den Boden der Ringnut 135 mündet. Dadurch wird eine Verbindung zwischen dem Anschlussstutzen 147 und der inneren Bestrahlungskammer 109 hergestellt. Der Flansch 116 besitzt zusätzlich einen axial verlaufenden Entlüftungskanal 149, der die äussere Bestrahlungskammer 111 mit einem Entlüftungsventil 150 an der Aussenseite des Flansches 116 verbindet.

Das Verschlussteil 104 besteht aus einer Platte 151 mit einem zentralen Anschlussstutzen 152. Der Innenfläche der Platte 151 liegt ein Ring 153 auf, der umfangsmässig der Innenwandung des Aussenmantels 102 anliegt.

Der Durchfluss durch den Dreikammer-Photoreaktor 100 erfolgt zwischen den Anschlussstutzen 147 und 152 durch die Bestrahlungskammern 109, 110 und 111, wobei die Bestrahlungskammern 110 und 111 durch die Durchtrittsöffnungen 108 in der Wand des einseitig geschlossenen Quarzglasrohres 107 miteinander kommunizieren. Zur Erzeugung eines gleichförmigen Strömungsprofils sind ringförmige Lochplatten 154, 155 vorgesehen. Die Lochplatte 154 ist an dem Steg 139 des zweiten Axialteils 125 des ersten Verschlussteils 103 befestigt und wirkt auf die die innere Bestrahlungskammer 109 durchsetzende Strömung ein. Die Lochplatte 155 liegt dem der Innenfläche der Platte 151 des zweiten Verschlussteils 104 aufliegenden Ring 153 an und wirkt auf die die äussere Bestrahlungskammer 111 durchsetzende Strömung ein; an ihrer Innenkante liegt das Quarzglasrohr 107 an, das dadurch an seinem geschlossenen Ende zusätzlich geführt ist. Die Lochplatten 154, 155 bestehen aus Material, das gegen UV-Strahlung und gegen das durchströmende Medium beständig ist und selbst keine Fremd- oder Schadstoffe an das Medium abgibt (rostfreier Stahl, beschichtete Metalle, Kunststoff, Keramik, Quarz, Glas). Die Weite der Löcher ist so gross, dass die Strömung nicht wesentlich behindert ist, jedoch ein über die Durchtrittsfläche gleichmässiges Strömungsprofil erzeugt wird. Dazu können die Löcher auch durch Öffnungen anderer geeigneter Gestalt wie Schlitze ersetzt werden.

Für den Dauerberieb des Dreikammer-Photoreaktors 100 spielt die Durchströmungsrichtung kaum eine Rolle. Wesentliche Unterschiede können sich jedoch beim Anlaufen des Betriebes ergeben. Bei wiederholten Unterbrechungen im Betrieb kann es erwünscht sein, schon nach kürzester Anlaufzeit Medium des geforderten Reinheits- bzw. Entkeimungsgrades zu erhalten. Dann ist es zweckmässig, das Medium über den Anschlusstutzen 152 von der äusseren Bestrahlungskammer 111 durch die innere Bestrahlungskammer 109 zum Anschlusstutzen 147 strömen zu lassen. Mit der gleichen Durchströmungsrichtung kann in Fällen von Niederschlagsbildung erreicht werden, dass der störende Effekt zunächst auf die äusseren Bestrahlungskammern beschränkt bleibt und nicht zu schnell das Gesamtergebnis in Frage stellt. Aus Gründen der Lampenkühlung wird man im allgemeinen jedoch die Strömungsrichtung von innen nach aussen bevorzugen, ebenso in Fällen der Begasung.

Der in Fig. 9 dargestellte Dreikammer-Photoreaktor 100 besitzt eine innere Bestrahlungskammer 109 mit einer Schichtdicke von 0.8 cm, eine mittlere Bestrahlungskammer 110 mit einer Schichtdicke von 1 cm und eine äussere Bestrahlungskammer 111 mit einer Schichtdicke von 3.4 cm. Der Aussendurchmesser des Hüllrohres 105 beträgt 4 cm, die Wandstärke der Quarzglasrohre 106 und 107 beträgt jeweils 0.4 cm, und die Transmission des Quarzglases bei 254 nm ist bei dieser Dicke T (0.4 cm) = 0.92. Im Hüllrohr 105 befindet sich eine Quecksilberniederdruck-Quarzlampe (G 36 T g; General Electric) von 75 cm effektiver Bogenlänge, deren Strahlungsfluss auf dieser Länge eine Leistung von 11 W UV-254 nm an das Medium an der durchstrahlten Innenfläche des Hüllrohres 105 abgibt. Zu Vergleichszwecken sind die in der folgenden Tabelle 5 angegebenen Werte auf einen Strahlungsfluss von 15 W UV-254 nm über eine effektive Länge der Einstrahlungsfläche von 1 m bei der Bestrahlungskammer 109 normiert. Die folgende Tabelle zeigt in analoger Weise wie die vorstehende Tabelle 4 die Durchflüsse Q-40 (m³/h) des Dreikammer-Photoreaktors 100 mit einer Gesamtschichtdicke von 5.2 cm und von Einkammer-Photoreaktoren mit einer Schichtdicke d = 1 cm bzw. d = 5.2 cm für Medien mit Transmissionsfaktoren von T (1 cm) = 0.9 bis 0.1, sowie die Steigerungsfaktoren F der Durchflüsse Q-40 des Dreikammer-Photoreaktors 100 gegenüber den vorgenannten Einkammer-Photoreaktoren.

Tabelle 5

Leistungsvergleich des Dreikammer-Photoreaktors 100 mit Einkammer-Photoreaktoren von 1 cm bzw. 5.2 cm Schichtdicke für Medien verschiedener Transmissionsfaktoren

| T (1 cm) | | 0.9 | 0.8 | 0.7 | 0.6 | 0.5 | 0.4 | 0.3 | 0.2 | 0.1 |
|---|---|---|---|---|---|---|---|---|---|---|
| Q-40 Drei-K. 100 | m³/h | 3.53 | 2.46 | 1.75 | 1.26 | 0.93 | 0.49 | 0.48 | 0.32 | 0.16 |
| Q-40 Ein-K. (d = 1 cm) | m³/h | 1.00 | 0.89 | 0.78 | 0.67 | 0.56 | 0.44 | 0.33 | 0.22 | 0.11 |

Tabelle 5 (Fortsetzung)
Leistungsvergleich des Dreikammer-Photoreaktors 100 mit Einkammer-Photoreaktoren von 1 cm bzw. 5.2 cm Schichtdicke für Medien verschiedener Transmissionsfaktoren

| T (1 cm) | | 0.9 | 0.8 | 0.7 | 0.6 | 0.5 | 0.4 | 0.3 | 0.2 | 0.1 |
|---|---|---|---|---|---|---|---|---|---|---|
| Q-40 Ein-K. (d = 5.2 cm) | m³/h | 2.59 | 1.40 | 0.70 | 0.31 | 0.12 | 0.04 | 0.01 | $2 \cdot 10^{-3}$ | $2 \cdot 10^{-5}$ |
| F (d = 1 cm) | | 3.52 | 2.76 | 2.24 | 1.89 | 1.67 | 1.11 | 1.43 | 1.43 | 1.43 |
| F (d = 5.2 cm) | | 1.36 | 1.75 | 2.50 | 4.01 | 7.63 | 12.80 | 57.30 | | |

15 W UV-254 nm in axialer Position; Hüllrohr $D_i$ = 4 cm.

Es ergibt sich aus der Tabelle 5, dass die Leistung des Einkammer-Photoreaktors mit der Schichtdicke d = 5.2 cm für T (1 cm) = 0.7 einen Durchfluss Q-40 = 0.78 m³/h aufweist; bei ebenfalls 4 cm Hüllrohraussendurchmesser und bei gleichem Transmissionsfaktor erreicht die Leistung des Einkammer-Photoreaktors maximal einen Wert von Q-40 (max) = 1 m³/h bei der Schichtdicke d = 2 cm. Die dreifache Unterteilung in dem in Fig. 9 dargestellten Dreikammer-Photoreaktor 100 ergibt dagegen einen Durchfluss Q-40 = 1.75 m³/h, so dass selbst gegenüber der Optimalleistung des Einkammer-Photoreaktors der Steigerungsfaktor immer noch 1.75 beträgt. Dieses Ergebnis wird erzielt, obwohl ein Teil der von der Strahlungsquelle ausgehenden UV-Strahlung von dem Quarzglas absorbiert wird, aus dem die Quarzrohre 106 und 107 bestehen (in der Berechnung berücksichtigt).

Der in Fig. 9 dargestellte Dreikammer-Photoreaktor 100 wird bevorzugt in allen solchen Fällen angewendet, in denen auch bei relativ niedrigen Transmissionsfaktoren hohe Entkeimungsgrade erzielt werden sollen, und ist daher nicht auf die Entkeimung von Trinkwasser oder dergleichen beschränkt.

Wie Tabelle 5 zeigt, ist der Einkammer-Photoreaktor nur im Bereich niedriger Schichtdicken, wie bei d = 1 cm, für den Bereich der Transmissionfaktoren T (1 cm) = 0.9 bis 0.3 anpassungsfähig, aber dies auf Kosten der Leistung. Der Einkammer-Photoreaktor zeigt bei einer Schichtdicke im Bereich von 5 cm bereits bei T (1 cm) = 0.7 einen so erheblichen Leistungsabfall, dass Medien mit noch niedrigeren Transmissionsfaktoren für eine wirtschaftliche Entkeimung oft nicht mehr infrage kommen. Dagegen zeigt der in Fig. 9 beschriebene Dreikammer-Photoreaktor 100, siehe Zeile 1 in Tabelle 5, im Bereich der Transmissionsfaktoren T (1 cm) = 0.9 bis 0.1 überlegene Leistung und Anpassungsfähigkeit. Der Dreikammer-Photoreaktor 100 nach Fig. 9 eignet sich für den Gesamtbereich der Trinkwasserentkeimung, erreicht aber auch noch den Bereich biologisch vorgeklärter Abwässer mit Transmissionsfaktoren T (1 cm) zwischen 0.6 und 0.25, und damit auch den von Zuckerlösungen, farblosem Essig, leichten Weinen. Der durch Fig. 9 beschriebene Dreikammer-Photoreaktor 100 ist auch für Spezialzwecke, z.B. die Wasserreinigung mit wesentlich erhöhten Strahlungsdosen gut geeignet.

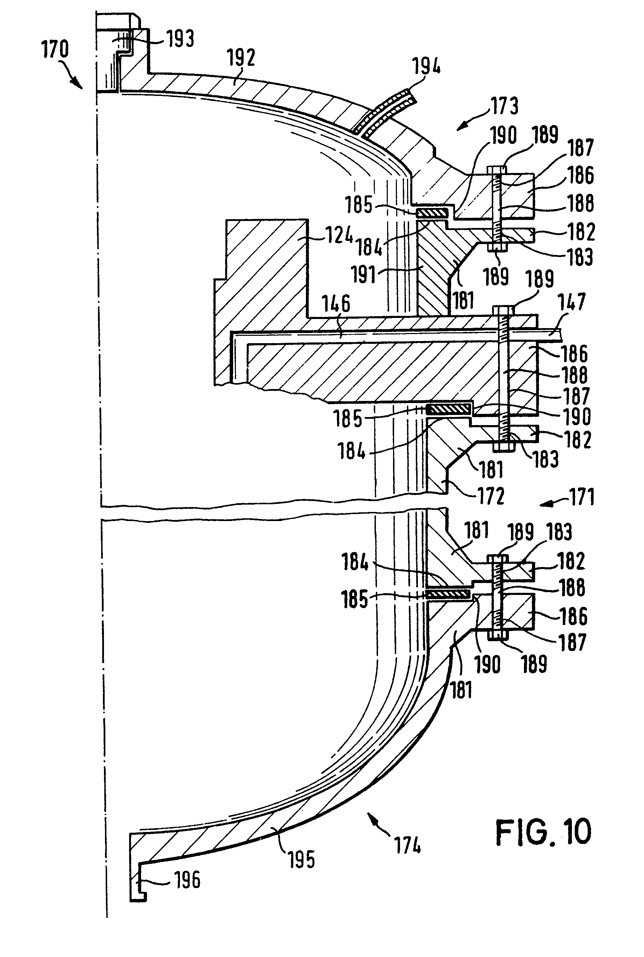

Fig. 10 zeigt eine Modifikation des Dreikammer-Photoreaktors 100 mit einer Druckausgleichseinrichtung. Dabei sind nur die gegenüber dem Dreikammer-Photoreaktor 100 geänderten Teile entsprechend Fig. 9 dargestellt und mit besonderen Bezugszeichen versehen.

Der Durchflussreaktor 171 nach Fig. 10 besteht aus einem Aussenrohr 172, aus einem ersten Verschlussteil 173 und einem zweiten Verschlussteil 174. Der (nicht gezeigte) Strahler 24 und die ebenfalls nicht gezeigten Quarzglasrohre 105, 106, 107 sind wie bei dem Durchflussreaktor 101 ausgebildet und angeordnet.

Der Aussenmantel 172 ist zur Verbindung mit den Verschlussteilen 173, 174 an beiden Enden mit Ringflanschen 182 versehen, die längs ihres inneren Umfangs verlaufende Verstärkungen 181 und längs ihres äusseren Umfangs verteilte Bohrungen 183 aufweisen. An der Aussenseite der Ringflansche 182 befinden sich Erhöhungen 184, die mit Dichtungen 185 in Ausnehmungen 190 an den jeweiligen Gegenflanschen 186 zusammenwirken. Die Gegenflansche 186 der Verschlussteile 173, 174 besitzen längs ihres inneren Umfangs verlaufende Verstärkungen 181 und längs ihres äusseren Umfangs verteilte Bohrungen 187, deren Zahl und Durchmesser den Bohrungen 183 in den Ringflanschen 182 des Aussenmantels 172 entspricht. Der Aussenmantel 172 und die Verschlussteile 173, 174 sind so angeordnet, dass die Bohrungen 183 und 187 fluchten, so dass sie durch Gewindebolzen 188, die sich durch die Bohrungen 183 und 187 erstrecken, und Muttern 189 fest und druckdicht miteinander verbunden sind.

Das Verschlussteil 173 ist an dem Gegenflansch 186 wie das Verschlussteil 103 mit Axialteilen versehen, von denen nur das Axialteil 124 in Andeutung gezeigt ist. Diese Axialteile sind identisch mit den Axialteilen 124, 125 des Durchflussreaktors 101 und dienen wie diese der Halterung von Quarzglasrohren 105, 106, 107; diese Teile sind daher in Fig. 10 nicht im einzelnen dargestellt. Wie der Flansch 116 besitzt auch der Gegenflansch 186 zwei in seiner Umfangsfläche mündende, diametral gegenüberliegende Radialkanäle 146, die in Anschlustutzen 147 enden.

An der dem Aussenrohr 172 abgekehrten Seite trägt der Gegenflansch 186 einen damit fest verbundenen oder aus einem Stück gebildeten Ansatz 191, an den über einen Ringflansch 182 ein gerundeter Deckel 192 mit einem Gegenflansch 186 in der vorstehend bereits beschriebenen Weise druckdicht angeflanscht ist. Der Deckel 192 weist eine zentrale, druckdichte, hochspannungs- und überschlagssichere Durchführung 193 für den Anschluss des (nicht gezeigten) Strahlers 24 auf. Ein Anschlusstutzen 194 ist zum Anschluss an einen Barostaten vorgesehen, der handelsüblich ausgebildet ist und daher hier nicht weiter im einzelnen beschrieben wird.

Der Verschlussteil 174 besteht aus einem gerundeten Deckel 195 mit einem zentralen Anschlusstutzen 196 und mit einem Gegenflansch 186 zur Verbindung mit dem anderen Ringflansch 182 des Aussenrohres 172 in der vorstehend bereits beschriebenen Weise. Innerhalb des Deckels 195 stützt sich ein hier nicht dargestellter Ring 153 ab, dem wie im Durchflussreaktor 101 eine Lochplatte 155 aufliegt.

Im Betrieb wird von dem Barostaten über den Anschlusstutzen 194 ein Druckgas auf den Durchflussreaktor 171 gegeben, vorzugsweise ein Inertgas wie Stickstoff, Argon oder Kohlendioxid. Durch den Barostaten wird ein Druck erzeugt und aufrechterhalten, der dem Innendruck des Durchflussreaktors 171 gleich ist. Dadurch wird vermieden, dass an den Quarzglasrohren 105, 106, 107 Druckdifferenzen auftreten, die zu mechanischen Spannungen und zum Bruch der Quarzglasrohre führen können.

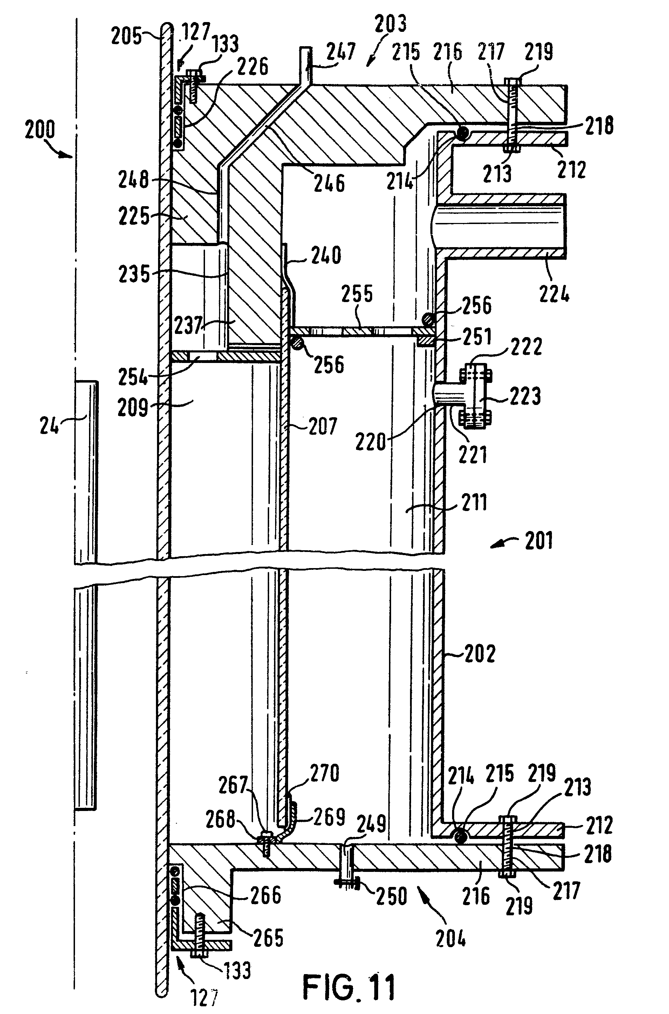

Fig. 11 zeigt eine weitere Ausbildung eines Mehrkammer-Photoreaktors, der sich von dem Dreikammer-Photoreaktor 100 im wesentlichen durch die Zahl der Bestrahlungskammern und durch die Ausbildung des Hüllrohres unterscheidet. In Fig. 11 ist ein Zweikammer-Photoreaktor 200 in gleicher Darstellung wie der Dreikammer-Photoreaktor 100 in Fig. 9 gezeigt.

Ein Durchflussreaktor 201 ist von einem strahlungsundurchlässigen Aussenmantel 202, von einem ersten Verschlussteil 203 und einem zweiten Verschlussteil 204 und von einem strahlungsdurchlässigen inneren Hüllrohr 205 gebildet, das von beiden Verschlussteilen 203 und 204 gehaltert ist. Das innere Hüllrohr 205 ist ein beidseitig offenes Quarzglasrohr. Der Durchflussreaktor 201 ist durch ein Quarzglasrohr 207, dessen Enden an den Verschlussteilen 203 bzw. 204 gehaltert sind, in zwei Bestrahlungskammern 209, 211 unterteilt.

Der Aussenmantel 202 ist zur Verbindung mit den Verschlussteilen 203, 204 an beiden Enden mit Ringflanschen 212 versehen, die längs ihres Umfangs verteilte Bohrungen 213 aufweisen. An der Aussenseite der Ringflansche 212 befinden sich Ausnehmungen 214 zur Aufnahme von abdichtenden O-Ringen 215. Die Verschlussteile 203, 204 tragen Flansche 216 mit längs ihres Umfangs verteilten Bohrungen 217. Der Aussenmantel 202 und die Verschlussteile 203, 204 werden durch Gewindebolzen 218, die sich durch die Bohrungen 213 und 217 erstrecken und durch Muttern 219 gesichert sind, fest und abdichtend miteinander verbunden.

Der Aussenmantel 202 ist wie der Aussenmantel 102 des Dreikammer-Photoreaktors 100 nach Fig. 9 zu Beobachtungs- oder Kontrollzwecken mit einer Öffnung 220 und einem Tubus 221 mit Ringflansch 222 und Deckel 223 versehen. Weiterhin trägt der Aussenmantel 202 nahe dem Ende, das dem Verschlussteil 203 benachbart ist, einen seitlichen Anschlusstutzen 224. Der Aussenmantel 202, die Verschlussteile 203, 204 und die Quarzglasrohre 205, 207 bestehen aus dem gleichen Material wie die entsprechenden Teile des Dreikammer-Photoreaktors 100.

Die Verschlussteile 203, 204 sind allgemein ringförmig ausgebildet und haben einen Innendurchmesser, der eng an den Aussendurchmesser des Hüllrohres 205 angepasst ist. Das Verschlussteil 203 besitzt ein Axialteil 225, das sich von dem Flansch 216 her an dessen Innenseite in das Innere des Durchflussreaktors 201 erstreckt und zur Halterung des Hüllrohres 205 bzw. des Quarzglasrohres 207 an einem Ende des Durchflussreaktors 201 dient. An seinem Aussenende ist das Verschlussteil 203 mit einer Gegenbohrung 226 versehen, in die eine Stopfbuchspackung 127 eingesetzt ist, die mit Schrauben 133 an der Aussenfläche des Verschlussteils 203 befestigt ist und das Hüllrohr 205 an diesem Ende des Durchflussreaktors 201 fest und abdichtend haltert. An seinem inneren Ende ist das Axialteil 225 mit einer ringförmigen Ausnehmung 235 versehen, die nach aussen von einem ringförmigen Steg 237 begrenzt ist. Das Axialteil 225 hat einen Aussendurchmesser, der eng an den Innendurchmesser des Quarzglasrohres 207 angepasst ist, so dass dessen eines Ende auf den Axialteil 225 aufgeschoben ist. Eine Dichtmanschette 240, die gegebenenfalls durch Ligaturen nach Art von Schlauchschellen gesichert ist, umgibt den freien Teil des Axialteils 225 und das auf den übrigen Teil aufgeschobene Ende des Quarzglasrohres 207. Dadurch wird dieses Ende des Quarzglasrohres 207 fest und abdichtend an dem Verschlussteil 203 gehalten.

Das Verschlussteil 203 besitzt einen in seiner Aussenfläche mündenden Kanal 246, der in einem Anschlusstutzen 247 endet. Der Kanal 246 ist an seinem inneren Ende mit einem Axialkanal 248 verbunden, der durch den Axialteil 225 hindurch verläuft und in den Boden der ringförmigen Ausnehmung 235 mündet. Dadurch wird eine Verbindung zwischen dem Anschlusstutzen 247 und der inneren Bestrahlungskammer 209 hergestellt.

Das Verschlussteil 204 besitzt ein Axialteil 265, das sich von dem Flansch 216 her an dessen Innenseite dem Durchflussreaktor 201 abgekehrt erstreckt und zur Halterung des Hüllrohres 205 am anderen Ende des Durchflussreaktors 201 dient. An seinem Aussenende ist das Verschlussteil 204 mit einer Gegenbohrung 266 versehen, in die eine Stopfbuchspackung 127 eingesetzt ist, die mit Schrauben 133 an der Aussenfläche des Verschlussteils 204 befestigt ist und das Hüllrohr 205 an diesem Ende des Durchflussreaktors 201 fest und abdichtend haltert. An seiner Innenfläche ist an dem Verschlussteil 204 mit Schrauben 267 ein Ring 268 befestigt, von dem kranzartig nach aussen gewölbte Blattfedern 269 vorspringen, zwischen denen eine das andere Ende des Quarzglasrohres 207 umgebende Schutzmanschette 270 geführt ist. Das Verschlussteil 204 besitzt einen axial verlaufenden Entleerungskanal 249, der die äussere Bestrahlungskammer 211 mit einem Entleerungsventil 250 an der Aussenseite des Flansches 216 verbindet.

Der Durchfluss durch den Zweikammer-Photoreaktor 200 erfolgt zwischen den Anschlussstutzen 224 und 247 durch die Bestrahlungskammern 209 und 211, die durch die (nicht dargestellten) Zwischenräume zwischen den von der Innenfläche des Verschlussteils 204 in den Durchflussreaktor 201 vorspringenden Blattfedern 269 miteinander kommunizieren. Zur Erzeugung eines gleichförmigen Strömungsprofils sind Lochplatten 254, 255 vorgesehen, die wie bei dem Dreikammer-Photoreaktor 100 ausgebildet sind. Die Lochplatte 254 ist an dem von der ringförmigen Ausnehmung 235 vorspringenden Steg 237 des Axialteils 225 vom Verschlussteil 203 befestigt und wirkt auf die die innere Bestrahlungskammer 209 durchsetzende Strömung ein. Die Lochplatte 255 liegt einem an der Innenfläche des Aussenmantels 202 nahe dem Stutzen 224 befestigten Ring 251 an, der damit auch aus einem Stück gebildet sein kann; innenseitig liegt sie dem Ende der Dichtmanschette 240 an. Die Lochplatte 255 ist durch Sicherungsringe 256 gegen eine Verschiebung gesichert; sie wirkt auf die die äussere Bestrahlungskammer 211 durchsetzende Strömung ein.

Der in Fig. 11 dargestellte Zweikammer-Photoreaktor 200 besitzt eine innere Bestrahlungskammer 209 mit einer Schichtdicke d = 2.4 cm und eine äussere Bestrahlungskammer 211 mit einer Schichtdicke d = 4.6 cm. Der Aussendurchmesser des Hüllrohres 205 beträgt 7.2 cm, die Wandstärke der Quarzglasrohre 205 und 207 beträgt jeweils 0.4 cm, und die Transmission des Quarzglases bei 254 nm ist bei dieser Dicke T (0.4 cm) = 0.92. Im Hüllrohr 205 befindet sich eine antimondotierte Xenon-Hochdrucklampe (Original Hanau Quarzlampen GmbH, Hanau), deren Strahlungsfluss im Bereich von 260 bis 280 nm bei einer effektiven Länge von 80 cm der Einstrahlungsfläche bei der Bestrahlungskammer 209 eine Leistung von 10 W an das Medium an der durchstrahlten Innenfläche des Hüllrohres 205 abgibt. Die folgende Tabelle 6 zeigt in analoger Weise wie die vorstehende Tabelle 5 die Durchflüsse Q-40 des Zweikammer-Photoreaktors 200 mit einer Gesamtschichtdicke d = 7 cm und eines Einkammer-Photoreaktors gleicher Schichtdicke für Medien mit Transmissionsfaktoren von T (1 cm) = 0.95 bis 0.6 sowie die Steigerungsfaktoren F der Durchflüsse Q-40 des Zweikammer-Photoreaktors 200 gegenüber dem vorgenannten Einkammer-Photoreaktor.

Tabelle 6
Leistungsvergleich des Zweikammer-Photoreaktors 200 mit einem Einkammer-Photoreaktor gleicher Gesamtschichtdicke (d = 7 cm) für Medien verschiedener Transmissionsfaktoren

| T (1 cm) | | 0.9 | 0.8 | 0.7 | 0.6 |
|---|---|---|---|---|---|
| Q-40 Zwei-K. 200 | m³/h | 27.8 | 16.4 | 9.8 | 5.9 |
| Q-40 Ein-K. | m³/h | 20.18 | 8.85 | 3.47 | 1.18 |
| F | | 1.38 | 1.85 | 2.85 | 5.0 |

100 W UV-260 bis 280 nm in axialer Position;
Hüllrohr $D_i$ = 7.2 cm.

Die Daten in der Tabelle sind für die Transmission des Quarzglases bei 254 nm angegeben, um so den Vergleich mit den Durchflusswerten Q-40 der gleichen Photoreaktoren bei Verwendung von Quecksilberniederdruck-Quarzlampen zu erleichtern. Die Transmission des Quarzglases ist im Bereich von 260 bis 280 nm jedoch höher, woraus sich eine Erhöhung der in der Tabelle angegebenen Q-40-Werte ergibt.

Der Zweikammer-Photoreaktor 200 ist bei einer Schichtdicke der inneren Bestrahlungskammer 209 von d = 2.4 cm und der äusseren Bestrahlungskammer 211 von d = 4.6 cm entsprechend einer Gesamtschichtdicke von d = 7 cm für hohe Durchflüsse Q-40 in dem für die Trinkwasserentkeimung wesentlichen Bereich der Transmissionsfaktoren T (1 cm) = 0.7 vorgesehen. Der Leistungsvergleich in Tabelle 6 zeigt, dass bereits bei Verwendung von zwei Bestrahlungskammern im Bereich zwischen T (1 cm) = 0.85 bis 0.7 Steigerungsfaktoren von 1.55 erreicht werden. Wegen der angestrebten Durchflüsse Q-40 wird hier auf mehr Bestrahlungskammern verzichtet; aus dem gleichen Grunde darf der Aussendurchmesser des Hüllrohres 205 nicht zu klein gewählt werden. Der Zweikammer-Photoreaktor 200 ist insbesondere für Zwecke der Wasserentkeimung in der Getränkeindustrie sowie für die UV-Desinfektion in der Trinkwasserversorgung geeignet.

Für den Dauerbetrieb des Zweikammer-Photoreaktors 200 spielt die Durchströmungsrichtung kaum eine Rolle. Aus Gründen der Lampenkühlung wird man im allgemeinen die Strömungsrichtung von der inneren Bestrahlungskammer 209 durch die äussere Bestrahlungskammer 211 bevorzugen, ebenso im Falle der Begasung. Wegen der hohen Durchflüsse treten auch bei Unterbrechungen im Betrieb des Zweikammer-Photoreaktors 200 praktisch keine störenden Anlauferscheinugnen auf. Lediglich bei der Gefahr des Auftretens von Niederschlägen wird man gegebenenfalls die umgekehrte Durchströmungsrichtung wählen.

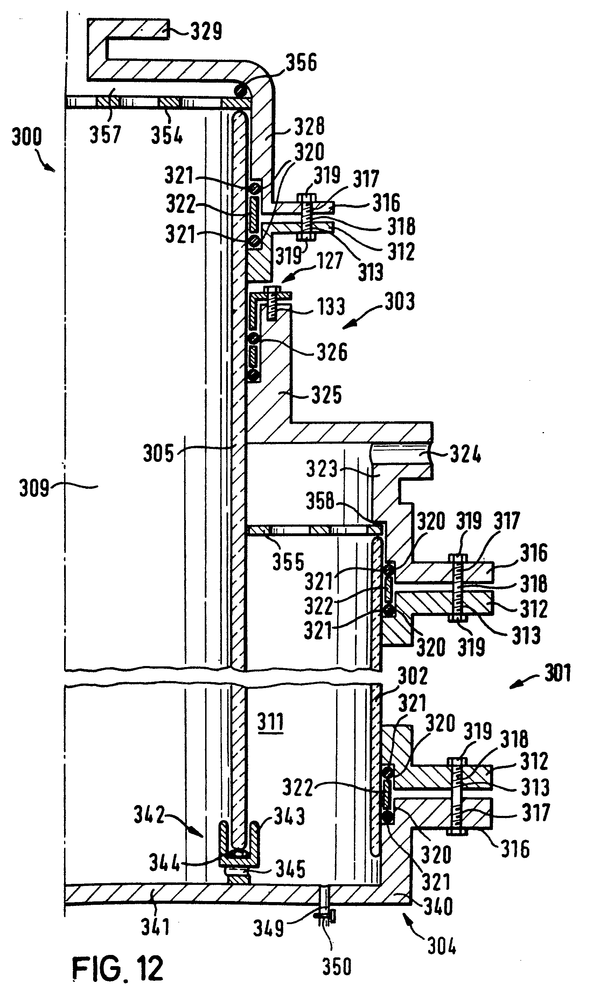

Eine weitere Ausführung eines Zweikammer-Photoreaktors ist in Fig. 12 dargestellt. Der Zweikammer-Photoreaktor 300 ist zur Bestrahlung von aussen mit einer (nicht gezeigten) Strahlungsquelle aus 14 Strahlern (Quecksilberniederdruck-Quarzlampen NN 30/89 Original Hanau Quarzlampen GmbH, Hanau) vorgesehen. Die Strahler befinden sich in einem konzentrisch zu einem Durchflussreaktor 301 angeordneten Reflektorsystem aus paraboloiden Reflektoren, deren jeder jeweils einem Strahler zugeordnet ist. Die gesamte Anordnung ist von einem strahlungsundurchlässigen Gehäuse umgeben, das auch die Vorschalt- und Bedienungselemente sowie die Überwachungseinrichtung für den Betrieb des Zweikammer-Photoreaktors 300 aufnimmt. Solche Gehäuse und Strahlungsquellen sind bekannt und im Handel erhältlich (WEDECO, Gesellschaft für Entkeimungsanlagen, Düsseldorf, Herford) und brauchen daher hier nicht im einzelnen beschrieben zu werden. Die Darstellung in Fig. 12 entspricht ansonsten der Darstellung des Dreikammer-Photoreaktors 100 in Fig. 9.

Der Durchflussreaktor 301 wird von einem strahlungsdurchlässigen Aussenrohr 302 aus Quarzglas, einer Halterung 303, einem Verschlussteil 304 und einem Innenrohr 305 aus Quarzglas gebildet. Das Innenrohr 305 teilt den Durchflussreaktor 301 in zwei Bestrahlungskammern 309, 311. Das Aussenrohr 302 ist über Ringflanschstücke 312, die nahe seinen Enden angeordnet sind, mit längs ihres Umfangs verteilten Bohrungen 313 mit der Halterung 303 bzw. dem Verschlussteil 304 verbunden. Die Halterung 303 bzw. das Verschlussteil 304 tragen Ringflansche 316 mit längs ihres Umfangs verteilten Bohrungen 317, deren Zahl und Durchmesser den Bohrungen 313 in den Ringflanschstücken 312 entspricht. Die Ringflanschstücke 312 und die Halterung 303 bzw. das Verschlussteil 304 sind so angeordnet, dass die Bohrungen 313 und 317 fluchten und die Teile durch mit Muttern 319 gesicherte Gewindebolzen 318 miteinander verbunden sind. Die Flanschteile 312 und 316 besitzen einen Innendurchmesser, der eng an den Aussendurchmesser des Aussenrohres 302 angepasst ist; innenseitig sind sie miteinander zugekehrten ringförmigen Ausnehmungen 320 versehen, gegen deren Boden O-Ringe 321 durch eine Führungshülse 322 gedrückt werden. Auf diese Weise wird das Aussenrohr 302 fest und abdichtend gehaltert.

Die Halterung 303, das Verschlussteil 304 und das Innenrohr 305 bestehen aus dem gleichen Material wie die entsprechenden Teile des Zweikammer-Photoreaktors 20.

Die Halterung 303 besitzt die Form eines axial abgestuften Ringes, der in seiner ersten Stufe 323 eng an den Aussendurchmesser des Aussenrohres 302 und mit einer Schulter 358 eng an den Innendurchmesser des Aussenrohres 302 angepasst und mit Anschlusstutzen 324 versehen ist; eine zweite Stufe 325 ist im Innendurchmesser eng an den Aussendurchmesser des Innenrohres 305 angepasst und trägt eine Gegenbohrung 326, in die eine Stopfbuchspackung 127 eingesetzt ist, die mit Schrauben 133 an der Ausssenfläche der Halterung 303 befestigt ist und das Innenrohr 305 fest und abdichtend in der Halterung 303 haltert. Oberhalb der Halterung 303 befindet sich ein weiteres Ringflanschstück 312, mit dem ein mit einem Ringflansch 316 versehenes Übergangsstück 328 entsprechend durch mit Muttern 319 gesicherte Gewindebolzen 318 unter Einlagerung von O-Ringen 321 fest und abdichtend verbunden ist. Das Übergangsstück 328 besitzt eine eng an den Aussendurchmesser des Innenrohres 305 angepasste lichte Weite; es verläuft ein Stück über das eine Ende des Innenrohres 305 hinaus und verengt sich dann zu einem Anschlusstutzen 329.

Das Verschlussteil 304 besteht aus einem den Flansch 316 tragenden axial verlaufenden Ring 340, der mit einer Platte 341, die den Durchflussreaktor 301 schliesst, fest verbunden ist oder aus einem Stück damit besteht. Die Platte 341 trägt an ihrer Innenseite einen aufgesetzten Ring 342, der fest damit verbunden ist oder aus einem Stück damit besteht und in einem hochstehenden Doppelring 343 von U-förmigem Querschnitt endet. Der Ring 342 verläuft unterhalb des Innenrohres 305 konzentrisch dazu; der Doppelring 343 ist an dessen Abmessungen angepasst, so dass das Innenrohr 305 an seinem anderen Ende in dem Doppelring 343 (unter Einlage eines elastischen Schutzrings 344) geführt ist. Der Ring 342 besitzt über seinen Umfang verteilte Durchtrittsöffnungen 345, über die die Bestrahlungskammern 309, 311 kommunizieren. Die Platte 341 ist mit einem axial verlaufenden Entleerungskanal 349 versehen, der die äussere Bestrahlungskammer 311 mit einem Entleerungsventil 350 an der Aussenseite der Platte 341 verbindet.

Der Durchfluss durch den Zweikammer-Photoreaktor 300 erfolgt zwischen den Anschlusstutzen 324 und 329 durch die Bestrahlungskammern 309 und 311. Zur Erzeugung eines gleichförmigen Strömungsprofils sind Lochplatten 354, 355 vorgesehen, die entsprechend dem Dreikammer-Photoreaktor 100 ausgebildet sind. Die Lochplatte 354 liegt innerhalb des Übergangsstückes 328, das mit der Halterung 303 verbunden ist, der Abschmelzkante des Innenrohres 305 auf und ist durch einen Sprengring 356 gesichert. Zwischen dem Anschlusstutzen 329 des Übergangsstücks 328 und der Lochplatte 354, die auf die die innere Bestrahlungskammer 309 durchsetzende Strömung einwirkt, ist ein Stauraum 357 ausgebildet.

Die Lochplatte 355 ist zwischen der Abschmelzkante an dem einen Ende des Aussenrohres 302 und der Schulter 358 gehalten, die in der ersten Stufe 323 der Halterung 303 ausgebildet ist, und wirkt auf die die äussere Bestrahlungskammer 311 durchsetzende Strömung ein.

Der in Fig. 12 dargestellte Zweikammer-Photoreaktor 300 besitzt eine äussere Bestrahlungskammer 311 mit einer Schichtdicke d = 2.5 cm und eine innere Bestrahlungskammer 309 mit einem Innendurchmesser von 9.2 cm. Der Aussendurchmesser des Aussenrohres 302 beträgt $D_a$ = 15.8 cm, die Wandstärke der Quarzglasrohre 302 und 305 beträgt jeweils 0.4 cm und die Transmission des Quarzglases bei 254 nm ist bei dieser Dicke T (0.4 cm) = 0.92. 14 in paraboloiden Reflektoren angeordnete Quecksilberniederdruck-Quarzlampen (NN 30/89 Original Hanau Quarzlampen GmbH, Hanau) umgeben den Durchflussreaktor 301 konzentrisch und geben über den Umfang des Aussenrohres 302 verteilt in das Medium eine mittlere Einstrahlungsleistung von 85 W UV-254 nm über eine effektive Länge der Bestrahlungskammer 311 von 79 cm. In der folgenden Tabelle 7 sind die Durchflüsse Q-40 des Zweikammer-Photoreaktors 300 und eines Einkammer-Photoreaktors mit analoger Aussenbestrahlung mit 6 Quecksilberniederdruck-Quarzlampen gleicher Art und mit einem Innendurchmesser von D = 7 cm angegeben, sowie die auf eine Leistung von 15 W UV-254 nm normierten Werte der Durchflüsse Q-40 bei Transmissionsfaktoren T (1 cm) von 0.9 bis 0.6. Die Tabelle zeigt ebenfalls die Steigerungsfaktoren F, die aus den normierten Durchflüsen Q-40 errechnet sind.

Tabelle 7
Leistungsvergleich des Zweikammer-Photoreaktors 300 mit einem Einkammer-Photoreaktor mit Aussenbestrahlung

| T (1 cm) | | 0.9 | 0.8 | 0.7 | 0.6 |
|---|---|---|---|---|---|
| Q-40 Zwei-K. 300 (14·11 W UV) | m³/h | 30.5 | 24.2 | 16.1 | 9.5 |
| Q-40 Zwei-K. 300 normiert auf 15W UV | m³/h | 2.97 | 2.38 | 1.57 | 0.91 |
| Q-40 Ein-K. (6·11 W UV) | m³/h | 5.91 | 5.84 | 5.65 | 4.72 |
| Q-40 Ein-K. (6·11 W UV) normiert auf 15 W | m³/h | 1.34 | 1.31 | 1.28 | 1.06 |
| F | | 2.21 | 1.85 | 1.23 | 0.85 |

Quecksilberniederdruck-Quarzlampen
11 W UV-254 nm in kranzförmiger Anordnung.
Zweikammer-Photoreaktor 300:
14 Strahler, $D_a$ = 15.8 cm
Einkammer-Photoreaktor:
6 Strahler, D = 7 cm.

Der Leistungsvergleich des Zweikammer-Photoreaktors 300 ist mit einem in der Praxis bewährten zylindrischen Einkammer-Photoreaktor mit Aussenbestrahlung durchgeführt, da solche Einkammer-Photoreaktoren wegen der Gefahr von Strömungskurzschlüssen nicht mit grösseren Durchmessern gebaut werden. Die Aussenbestrahlung bietet als Alternative zur Leistungssteigerung axialer Strahlungsquellen die Möglichkeit, erheblich gesteigerte Raum-Zeit-Ausbeuten, d.h. höhere Durchflüsse Q-40 bei gleichem Apparatevolumen, zu erzielen. Obgleich solche Photoreaktoren mit Aussenbestrahlung infolge der positiven Bestrahlungsgeometrie wesentlich weniger die Nachteile starker Gradienten der Bestrahlungsstärke im Reaktorquerschnitt aufweisen, bietet auch hier das Mehrkammer-Photoreaktor-Prinzip erhebliche Leistungssteigerungen.

Im praktischen Betrieb des Zweikammer-Photoreaktors 300 spielt die Durchströmungsrichtung keine wesentliche Rolle.

Die Anwendung des Zweikammer-Photoreaktors 300 für Reinigungszwecke ist in den folgenden Versuchsbeispielen dargestellt:

1. Beseitigung von Rest-Ozon aus Wasser
Ozonisiertes Wasser mit einem Rest-Ozongehalt von 0.3 g/m³ (0.3 ppm) wird mit einem Durchfluss von Q = 40 m³/h durch den Zweikammer-Photoreaktor 300 geleitet. Das in die innere Bestrahlungskammer 309 eintretende Wasser ist nach dem Austritt aus der äusseren Bestrahlungskammer 311 praktisch ozonfrei (<0.02 ppm); Nachweis durch Palinsches Reagens bzw. kolorimetrische Analyse (Diethyl-p-phenylendiamin und Kaliumjodid).

2. Entfernung von aromatischen Kohlenwasserstoffen aus Wasser
Eine Emulsion von ca. 10 g eines aromatischen Teeröls in 70 m³ Wasser entsprechend dem Wasserinhalt eines Schwimmbassins enthält ca. 0.13 mg/l Aromaten, die durch ihre charakteristische UV-Absorption nachgewiesen werden. Das Wasser wird durch eine Sandfilteranlage mit einer Förderleistung von 25 m³/h im Kreislauf geführt. Dabei ändert sich der Aromatengehalt (UV-Absorption) nicht. Wird der Sandfilteranlage ein Zweikammer-Photoreaktor 300 nachgeschaltet, so sind im Auslauf des Photoreaktors keine aromatischen Verunreinigungen mehr nachweisbar (UV-Absorption, 5 cm-Küvette).

Eine weitere Leistungssteigerung bei dem Zweikammer-Photoreaktor 300 ist erzielbar, wenn zugleich eine Innenbestrahlung vorgesehen wird. Ein Photoreaktor solcher Art wird auf einfache Weise durch Kombination der entsprechenden Elemente aus Fig. 11 und 12 erhalten, so dass sein Aufbau hier nicht im einzelnen geschildert zu werden braucht. Als innere Strahlungsquelle dient dabei eine antimondotierte Xenon-Hochdrucklampe, die zur Erhöhung der Bestrahlungsstärke auch einfach oder mehrfach gewendelt sein kann; als äussere Strahlungsquellen dienen Quecksilberdampflampen geeigneter Emissionsbereiche. Solche Strahler sind im Handel erhältlich und brauchen daher nicht im einzelnen dargestellt und erläutert zu werden.

Weitere Abwandlungen im Aufbau der Durchflussreaktoren 2, 21, 41, 101, 201, 301 ergeben sich daraus, dass dem Fachmann eine Reihe bekannter, teilweise anders ausgebildeter Halterungs- und Führungselemente für die den Reaktorraum unterteilenden Trennwände zur Verfügung stehen. Diese können anstelle der in Fig. 1 bis 12 dargestellten Einrichtungen Verwendung finden. In vielen Fällen genügt auch eine Ausführung mit nur einem seitlichen Anschlusstutzen 147, 224 oder 324.

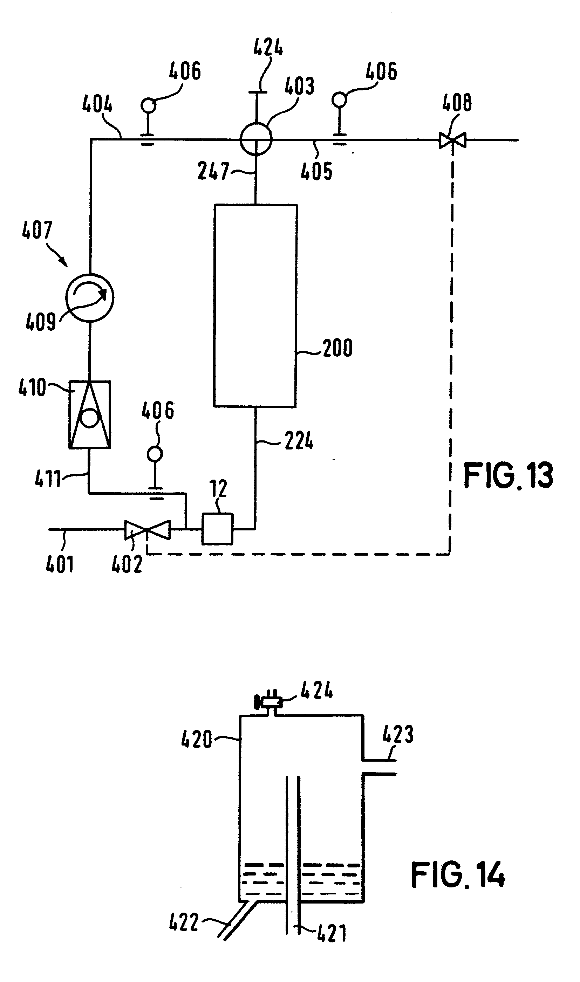

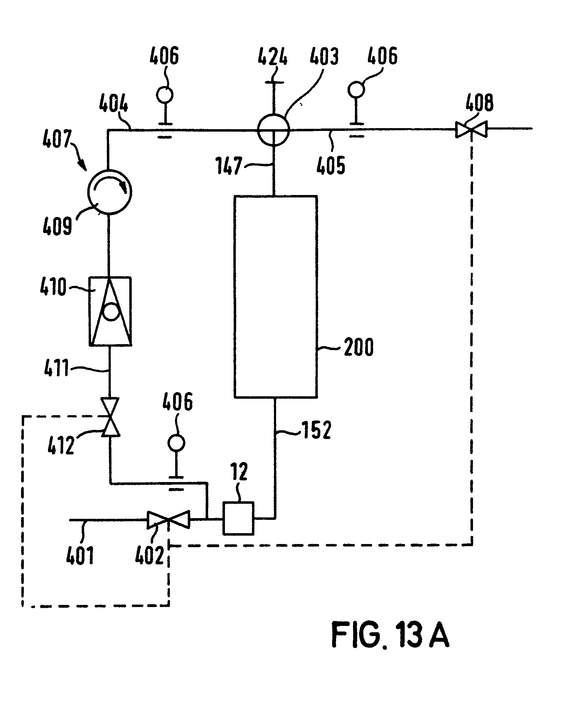

Ein Problem besteht in all den Fällen, in denen die Entnahme des bestrahlten Mediums Schwankungen unterliegt und sogar vorübergehend unterbrochen wird, gleichwohl aber eine konstante hohe Mindestleistung im Reinigungs- bzw. Entkeimungsgrade verlangt wird. In solchen Fällen wird ein Mehrkammer-Photoreaktor nach Art von Fig. 9 bis 12 im Rücklauf betrieben. Fig. 13 zeigt schematisch ein Fliessdiagramm für den Bestrahlungsbetrieb mit Rücklauf für den Dreikammer-Photoreaktor 200; es kann jedoch stattdessen auch ein Mehrkammer-Photoreaktor 20, 100 oder 300 eingesetzt werden. Auch lässt sich der ohnehin für einen Bestrahlungskreislauf vorgesehene Zweikammer-Photoreaktor 40 verwenden, der in dieser Betriebsweise bei Klimawäschern zum Einsatz gelangt, für die Durchlaufdesinfektion mit teilweisem Rücklauf jedoch weniger geeignet ist als die anderen genannten Mehrkammer-Photoreaktoren. Das Fliessdiagramm enthält den Dreikammer-Photoreaktor 200, dessen Anschlusstutzen 224 über eine Zufuhrleitung 401 und ein Zufuhrventil 402 an die Vorratsleitung des zu bestrahlenden Mediums angeschlossen ist. Der Anschlusstutzen 247 ist über einen Strömungsteiler 403 mit Entlüftungsventil 424 und Verbindungsleitungen 404, 405, die jede einen Durchflussanzeiger 406 tragen, mit einem Rücklauf 407 bzw. einem Entnahmeventil 408 verbunden. Dem Zufuhrventil 402 ist dabei ein Durchflussbegrenzer 12 nachgeschaltet. Der Rücklauf 407 besteht aus einer Rücklauf-Förderpumpe in Gestalt einer Einweg-Förderpumpe 409 mit konstanter Förderleistung, einem nachgeschalteten Rückschlagventil 410 und einer Verbindungsleitung 411 mit Durchflussanzeiger 406, die stromab von dem Ventil 402 in die Zufuhrleitung mündet. Der Rücklauf 407 kann anstelle der Einweg-Förderpumpe 409 auch eine in ihrer Förderleistung einstellbare Rücklauf-Förderpumpe enthalten; gegebenenfalls kann der Rücklauf 407 auch mit einer einstellbaren Strömungsdrossel ausgerüstet sein. Dabei ist das Gesamtvolumen des Rücklaufs 407 klein gegen das Volumen des jeweiligen Mehrkammer-Photoreaktors.

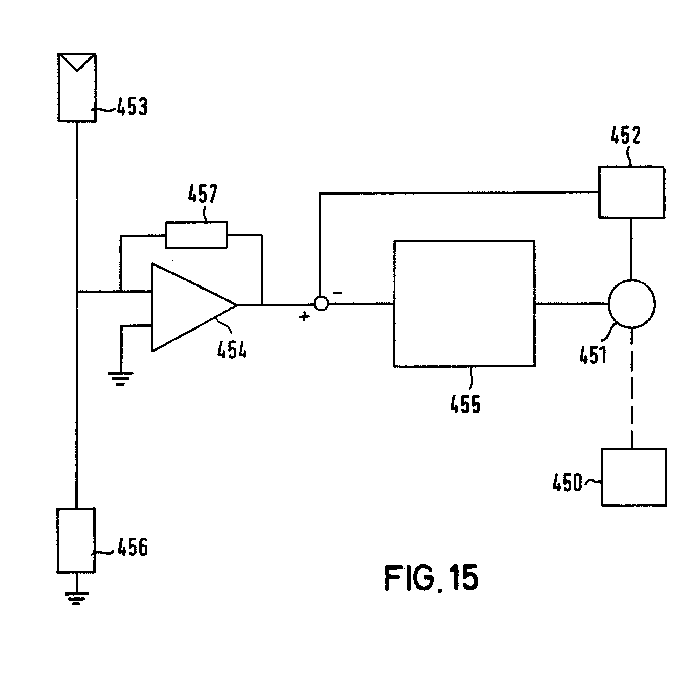

Der in Fig. 14 dargestellte Strömungsteiler 403 ist nach Art eines Drucküberlaufreglers aufgebaut. Die Schnittdarstellung nach Fig. 14 zeigt ein Gefäss 420, das oben mit einem Entlüftungsventil 424 versehen ist und dessen Eingangsstutzen 421 zum Anschluss an den Zweikammer-Photoreaktor 200 vorgesehen ist. Der Eingangsstutzen 421 ragt über den Boden des Gefässes 420 hinaus in dessen Inneres hinein. Vom Boden des Gefässes 420 aus verläuft ein erster Ausgang 422, der zum Anschluss an die Verbindungsleitung 404 zum Rücklauf 407 führt.

Ein zweiter Ausgang 423 ist deutlich oberhalb der Mündung des Eingangsstutzens 421 an dem Gefäss 420 angeordnet und dient zum Anschluss an die Verbindungsleitung 405 zum Entnahmeventil 408. Die in Fig. 13 und 14 dargestellte Anordnung arbeitet wie folgt, wobei angenommen ist, dass die Anlage mit dem Medium beschickt und entlüftet und dass die Ventile 402 und 408 zunächst geschlossen sind: Bei geschlossenem Entnahmeventil 408 und laufender Einweg-Förderpumpe 409 ergibt sich ein in sich geschlossener Kreislauf des Mediums, das über die Verbindungsleitungen 411, 401 und den Anschlusstutzen 224 des Zweikammer-Photoreaktors 200 in die Bestrahlungskammer 211 eintritt und diese nach Durchtritt durch die innere Bestrahlungskammer 209 über die Anschlusstutzen 247 wieder verlässt. Von dort gelangt es über den Eingangsstutzen 421 in das Innere des Gefässes 420 des Strömungsteilers 403, den es über den ersten Ausgang 422 verlässt, der über die Verbindungsleitung 404 eingangsseitig an die Einweg-Förderpumpe 409 angeschlossen ist.

Die Öffnung des Entnahmeventils 408 erfolgt synchron gekoppelt mit der Öffnung des Zufuhrventils 402, durch das dem Zweikammer-Photoreaktor 200 über die Zufuhrleitung 401 zu bestrahlendes Medium zugeführt wird. Die Kopplung geschieht dabei durch bekannte mechanische, elektrische, hydraulische, pneumatische Mittel oder dergleichen. Entsprechend dem zugeführten Volumen an zu bestrahlendem Medium wird nun bestrahltes Medium durch das geöffnete Entnahmeventil 408 aus dem Bestrahlungskreislaufsystem verdrängt. Da das zugeführte Medium bereits vor Eintrit in den Zweikammer-Photoreaktor 200 durch das im Rücklauf geführte bereits entkeimte Medium verdünnt wird, passiert nun ein Medium mit niedrigerer Eingangskeimzahl den Photoreaktor und eine niedrigere Endkeimzahl resultiert. Dabei ist zu berücksichtigen, dass durch die Rückführung bereits hochgereinigtes Material einer erneuten Bestrahlung unterworfen wird im Gemisch mit vorher unbestrahltem Medium, was im Ganzen eine Effizienzänderung bedeutet. Bei dieser Arbeitsweise muss daher jeweils ein niedrigerer Durchfluss als im Betrieb ohne Rücklauf eingestellt werden, und zwar richtet sich diese Erniedrigung auch nach dem Rücklaufverhältnis.

Um die Rücklaufbestrahlung mit grösserer Wirksamkeit zu betreiben, wird jedoch zweckmässiger das zu entkeimende Medium diskontinuierlich in geringer Menge hinzugegeben und hindurchgeführt. Dies geschieht durch chargenweise Verdrängung eines Grossteils des Reaktorinhalts bei gleichzeitigem Stop des Rücklaufbetriebes, gefolgt von einer Periode der Bestrahlung im Kreislauf, die je nach den gewünschten Dosen mehrere Umwälzungen des Reaktorvolumens betragen kann. Dazu ist im Rücklauf 407 ein weiteres gesteuertes Ventil 412 (siehe Fig. 13A) vorgesehen, das durch eines der vorgenannten Mittel synchron im Gegentakt mit dem Entnahmeventil 408 bzw. dem Zufuhrventil 402 gekoppelt ist. Die beiden zuletzt genannten gekoppelten Ventile bleiben so lange geöffnet, bis die vorgesehenen Portionen des unbestrahlten Mediums den Photoreaktor gefüllt und das bestrahlte Medium den Photoreaktor verlassen haben. Nach Schliessen der Ventile wird synchron das Ventil 412 im Rücklauf 407 geöffnet und die Bestrahlung im Kreislauf bis zur nächsten Beschickungsperiode vorgenommen. Eine kontinuierliche Entnahme des entkeimten Mediums kann dann dadurch erreicht werden, dass das Entnahmeventil 408 an einen Zwischenbehälter mit Niveauregelung und einer mit einem Durchflussbegrenzer ausgestatteten Entnahmestelle angeschlossen ist.

Am einfachsten lässt sich die diskontinuierliche Zufuhr des Mediums mit Hilfe einer gesteuerten Dosierpumpe durchführen, deren jeweilige Dosierportionen knapp unter dem Reaktorvolumen bleiben müssen. Das weitere gesteuerte Ventil 412 im Rücklauf 407 ist dabei durch eines der vorgenannten Mittel synchron im Gegentakt an die Dosierpumpe gekoppelt, so dass die Zufuhr des Mediums nur bei geschlossenem Rücklauf 407 erfolgt. Das Entnahmeventil 408 und das Zufuhrventil 402 können dann entfallen. Die Niveauregelung der vorerwähnten kontinuierlichen Entnahmevorrichtung mit Zwischenbehälter kann auch dazu benutzt werden, bei verändertem Bedarf innerhalb der Grenzen der Leistung der Apparatur die Periode der Dosierung und damit den Durchschnitt des Duchflusses zu variieren. Auf diese Weise besteht die Möglichkeit, beabsichtigte Erhöhungen der Dosis bei vollem Erhalt der Funktion eines gegebenen Photoreaktors zu erreichen.

Bei der Reinigung und Desinfektion durch UV-Bestrahlung nach dem geschilderten Verfahren der Rücklaufbestrahlung empfiehlt es sich, Mehrkammer-Photoreaktoren so zu betreiben, dass das Medium die Bestrahlungskammer mit dem geringsten Querschnitt und der höchsten Bestrahlungsstärke zuletzt durchläuft.

Mehrkammer-Photoreaktoren mit der einfachen Art des Rücklaufbetriebs eignen sich besonders zur Wasserentkeimung auf Seeschiffen. Das Verfahren der portionsweisen Rücklaufbestrahlung ist besonders für die Applikation hoher Dosen geeignet und damit zur Erzielung höchster Reinigungs- und Entkeimungsgrade.

Die erforderliche Sicherheit in der Erzielung des gewünschten Bestrahlungsergebnisses wird durch Durchflusssteuermittel erreicht, die dafür sorgen, dass eine bestimmte, maximal zulässige Durchströmungsgeschwindigkeit des Mediums in den Mehrkammer-Photoreaktoren nach Fig. 1 bis 12 nicht überschritten werden kann. Im einfachsten Fall genügt als Sicherheitselement eine Strömungsdrossel in der Zuleitung zu dem jeweiligen Durchflussreaktor. Bei wechselndem Eingangsdruck empfiehlt sich eine einstellbare Strömungsdrossel z.B. in Gestalt eines Ventils, jedoch wird vorzugsweise hier der zuverlässigere Durchflussbegrenzer 12 eingesetzt. Dessen Zwischenschaltung wird aus Sicherheitsgründen auch dann vorgenommen, wenn eine Pumpe mit einstellbarer Förderleistung verwendet wird, bei der die Förderleistung unmittelbar eingestellt und auch überwacht werden kann.

Die vorbeschriebenen Mehrkammer-Photoreaktoren sind mit üblichen, bekannten Überwachungseinrichtungen der eingangs genannten Art versehen. Dadurch wird sichergestellt, dass bei Abfall der Bestrahlungsstärke unter einen vorgegebenen Sollwert ein Alarm ausgelöst und die gesamte Bestrahlungsanlage abgeschaltet wird. Ausserdem nimmt der Strahlungsfluss der Strahler mit der Zeit ab. Wegen der eingangs diskutierten exponentiellen Abhängigkeit des Bestrahlungsergebnisses und damit auch der Leistung des Mehrkammer-Photoreaktors von der Bestrahlungsstärke ist für eine optimale Nutzung der von der Strahlungsquelle emittierten Strahlung eine ständige Anpassung der Durchströmungsgeschwindigkeit an die augenblickliche Bestrahlungsstärke notwendig. Dazu ist eine Förderpumpe 450 mit einstellbarer Förderleistung eingangsseitig an einen Durchflussreaktor 101, 201 oder 301 angeschlossen, und ihre Förderleistung wird durch eine Steuerung, die in Fig. 15 im Blockschaltbild dargestellt ist, entsprechend der jeweiligen Bestrahlungsstärke eingestellt. Die Steuerung besteht aus einem mit dem Pumpenmotor 451 verbundenen Tachogenerator 452 und einem strahlungsempfindlichen Detektor 453, der über einen Ableitungswiderstand 456 an Erde liegt und an dem Tubus 121, 221 eines Durchflussreaktors 101 bzw. 201 oder an dem Innenrohr 302 des Durchflussreaktors 301 (mit einer geeigneten Durchführung) angebracht ist und dessen Ausgangssignal einem Verstärker 454 anliegt. Die Ausgangssignale des Tachogenerators 452 und des Verstärkers 454 sind an dem Eingang eines Leistungsverstärkers 455 einander entgegengeschaltet, und die am Ausgang des Leistungsverstärkers 455 anliegende, verstärkte Differenzspannung dient zur Versorgung des Pumpenmotors 451. Auf diese Weise wird durch eine aus handelsüblichen Bauteilen aufgebaute Steuerung die Förderleistung der Pumpe 450 an die jeweilige Bestrahlungsstärke angepasst.

Bei den vorstehend geschilderten Mehrkammer-Photoreaktoren 100, 200, 300 sind die jeweiligen Bestrahlungskammern in bezug auf die Durchströmungsrichtung hintereinandergeschaltet. Diese Schaltung besitzt ihre besonderen Vorteile in der besseren Durchmischung und dem Durchgang des Mediums durch sämtliche Bestrahlungskammern des Photoreaktors. In besonderen Fällen kann jedoch auch eine Parallelschaltung von Bestrahlungskammern von Vorteil sein, und zwar dann, wenn Medien mit hohen Transmissionsfaktoren zu verarbeiten sind.

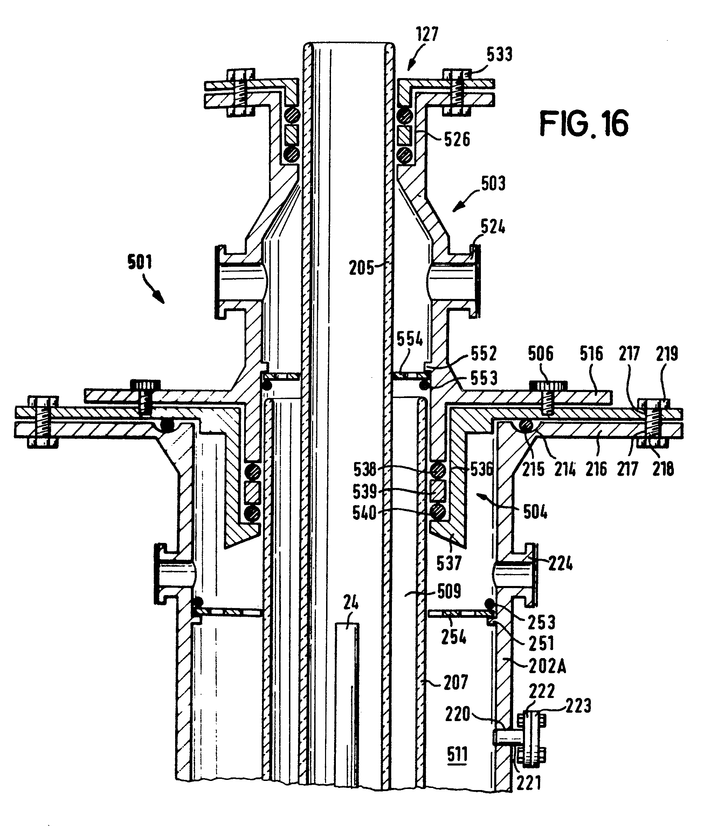

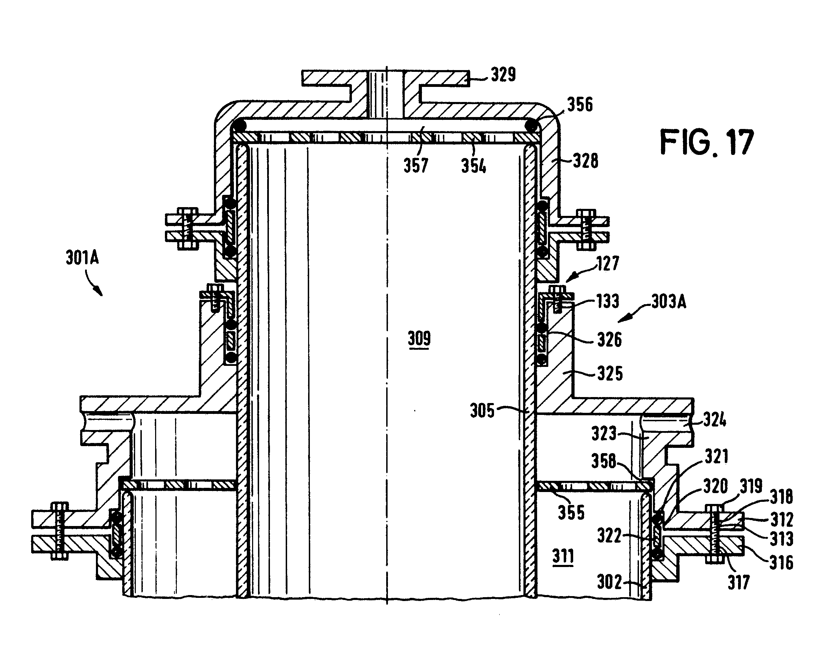

Durchflussreaktoren nach Art von Fig. 11 und 12 können leicht so abgeändert werden, dass die Bestrahlungskammern 209 und 211 bzw. 309 und 311 entsprechend Fig. 16 bzw. 17 zu paralleler Durchströmung geeignet sind. Die abgeänderte Ausführung des Zweikammer-Photoreaktors 200 besteht aus einem Durchflussreaktor 501, der eine UV-Strahlungsquelle 24 umgibt und im wesentlichen zwei Bestrahlungskammern 509 und 511 enthält, deren jede mit Einlass- und Auslassanschlüssen versehen ist. Fig. 16 zeigt einen Längsschnitt durch eine Hälfte des Durchflussreaktors 501, dessen andere Hälfte sehr ähnlich dazu spiegelbildlich ausgebildet ist.

Der Durchflussreaktor 501 besteht aus einem Aussenmantel 202A, der sich vom Aussenmantel 202 des Durchflussreaktors 201 nur dadurch unterscheidet, dass ein weiteres Paar einander gegenüberliegender Anschlussstutzen 224 nahe dem anderen, in Fig. 16 nicht dargestellten Ringflansch 212 vorhanden ist. Es ist jedoch nur eine Beobachtungsöffnung 220 vorgesehen, in die ein Tubus 221 mit einem Ringflansch 222 und einem Deckel 223 eingesetzt ist.

An beiden Enden ist der Durchflussreaktor 501 durch identisch ausgebildete Verschlussteile 503 mit einem Zwischenflanschglied 504 verschlossen, an dem die Verschlussteile 503 beispielsweise durch Schraubbolzen 506 befestigt sind, die sich durch die Flansche 516 der Verschlussteile 503 erstrecken. Die Zwischenflanschglieder 504 besitzen Flansche 216 mit Bohrungen 217, die nahe dessen Umfang über den Flansch 216 verteilt sind. Der Aussenmantel 202 und die Zwischenflanschglieder 504 werden fest und abgedichtet durch Bolzen 218 miteinander verbunden, die sich durch die Bohrungen 217 erstrecken und durch Muttern 219 gesichert sind, wobei in ringförmigen Ausnehmungen 214 Dichtringe 215 angeordnet sind.

Die Verschlussteile 503 sind im allgemeinen ringförmig aufgebaut und erstrecken sich axial von einem Aussenende, das eng an den Aussendurchmesser des Hüllrohres 205 angepasst ist, bis zu einem Innenende, das eng an den Aussendurchmesser des Quarzglasrohres 207 angepasst ist. Am Aussenende befindet sich eine Gegenbohrung 526, in die eine Stopfbuchspackung 127 eingesetzt ist, die mittels Bolzen 533 an flanschartigen Teilen befestigt sind und dazu dienen, das Hüllrohr 205 fest und abdichtend zu haltern. In einem Zwischenbereich zwischen den axialen Enden erweitern sich die Axialteile der Verschlussteile 503, um das Quarzglasrohr 207 aufzunehmen. Zwei diametral gegenüberliegende Anschlussstutzen 524 sind an dem erweiterten Axialteil angeordnet, um das zu bestrahlende Medium in die innere Bestrahlungskammer 509 einzubringen. Dicht an den Anschlussstutzen 524 ist an der Innenwand des erweiterten Axialteils eine Schulter 552 ausgebildet, der eine Lochplatte 554 anliegt, die durch einen Sicherungsring 553 gesichert ist. Das axiale Innenende des Verschlussteils 503 erstreckt sich jeweils über den Flansch 516 hinaus zu einem weiter unten erläuterten Zweck.

Jedes Zwischenflanschglied 504 ist ebenfalls im allgemeinen ringförmig ausgebildet und besteht aus einem Flanschteil und einem Axialteil 537, dessen Innendurchmesser eng an den Aussendurchmesser des Quarzglasrohres 207 angepasst ist. Das Axialteil 537 ist mit einer Gegenbohrung 536 versehen, die sich über eine solche Länge erstreckt, dass das axiale Innenende des Verschlussteiles 503 und eine Stopfbuchspackung aus zwei Dichtringen 538, 540 und einer Führungsbuchse 539 darin aufgenommen werden können. Diese Anordnung dient zusammen mit dem Flansch 516 des Verschlussteils 503, der durch Schraubbolzen 506 an dem Flansch des Zwischenflanschgliedes 504 befestigt ist, zur festen und abdichtenden Halterung des Quarzglasrohres 207 in ähnlicher Weise, wie das Hüllrohr 205 durch die Stopfbuchspackung 127 gehalten ist. Die Stirnseite des Axialteils 537 ist nach innen abgeschrägt, um die zentrierte Einführung des Quarzglasrohres 207 beim Zusammenbau des Durchflussreaktors 501 zu erleichtern.

Die abgeschrägten Stirnseiten der Axialteile 537 der Zwischenflanschglieder 504 erstrecken sich nicht bis in den Bereich der Anschlussstutzen 224 um sicherzustellen, dass dadurch der Durchfluss des Mediums durch die äussere Bestrahlungskammer 511 nicht behindert wird. Dicht an der anderen Seite der Anschlussstutzen 224 ist an der Innenwand des Aussenmantels 202A eine Ringschulter 251 ausgebildet, der eine Lochplatte 254 anliegt, die durch einen Sicherungsring 253 gehaltert wird.

Die Teile des Durchflussreaktors 501 bestehen aus dem gleichen Material wie die entsprechenden Teile des Durchflussreaktors 201.

Fig. 17 zeigt einen Längsschnitt durch eine Hälfte eines Durchflussreaktors 301A, der in Verbindung mit einer äusseren Strahlungsquelle verwendet wird. Die beiden Hälften des Durchflussreaktors 301A sind im wesentlichen spiegelbildlich zueinander ausgebildet und in ihrem Aufbau identisch mit dem Teil des Durchflussreaktors 301, der in Fig. 12 oberhalb der Bruchlinie dargestellt ist. Es ist daher an dieser Stelle keine weitere Erläuterung erforderlich, ausser dass zwei diametral gegenüberliegende Paare von Anschlussstutzen 324 die Anschlüsse für den Einlass und Auslass der Bestrahlungskammer 311 bilden, während die mittigen Anschlussstutzen 329 den Einlass und den Auslass der Bestrahlungskammer 309 bilden.

Eine Anwendung solcher Durchflussreaktoren findet sich in Verbindung mit Anlagen zur umgekehrten Osmose, die in zahlreichen Bereichen zur Herstellung reinen Wassers, z.B. bei der Trinkwassergewinnung aus Meerwasser, für Sonderzwecke von Kliniken, Elektroniklabors und pharmazeutischen Betrieben, sowie in der Nahrungsmittelindustrie eingesetzt wird. Für die umgekehrte Osmose sind verschiedene Typen von Membranen, oft auf der Basis organischer Materialien, gebräuchlich, die sich als anfällig gegen Bewuchs durch Mikroorganismen erwiesen haben, wodurch die Betriebsfähigkeit der Anlagen und die hygienische Qualität des erzeugten Wassers gefährdet werden. Aus Sicherheitsgründen wird oft der Umkehrosmose-Anlage eine UV-Entkeimung nachgeschaltet. Zweckmässig wird aber bereits das in die reversible Osmose eingeführte Medium einer UV-Entkeimung unterworfen, um so den Mikroorganismenbefall der Membranen von vornherein zu minimieren. Hier bietet der Zweikammer-Photoreaktor mit parallel geschalteten Bestrahlungskammern eine besonders günstige technische Lösung, um mittels eines Durchflussreaktors und einer Strahlungsquelle sowohl das Ausgangsmedium als auch das Produktwasser gleichzeitig zu entkeimen.

Zur Erhöhung der photochemischen Effizienz der Reinigung bzw. Entkeimung empfiehlt es sich, mindestens eines der parallel geschalteten Bestrahlungssysteme nach Art des Mehrkammer-Photoreaktors mit Serienschaltung auszubilden.

## Patentansprüche

1. Verfahren zur Reinigung, bei dem ein fliessfähiges Medium zur Einhaltung einer vorbestimmten Mindestdosis der ultravioletten Strahlung mit einem bestimmten Durchfluss durch einen Durchflussreaktor, der durch mindestens eine für ultraviolette Strahlung durchlässige Trennwand in mindestens zwei Bestrahlungskammern senkrecht zur Durchstrahlungsrichtung unterteilt ist, gefördert wird, und bei dem in den in bezug auf die durch die Strahlungsquelle bestimmte Durchstrahlungsrichtung hintereinander angeordneten Bestrahlungskammern durch das Medium in allen Bestrahlungskammern ein bestimmter Anteil und in der der Strahlungsquelle unmittelbar benachbarten Bestrahlungskammer ein Teil der in den Durchflussreaktor eintretenden ultravioletten Strahlung absorbiert wird, dadurch gekennzeichnet, dass das Medium in allen Bestrahlungskammern insgesamt nicht mehr als $(1-0.5^n) \cdot 100\%$ der in den Durchflussreaktor eintretenden Strahlung absorbiert, wobei n die Zahl der Bestrahlungskammern ist.

2. Verfahren nach Anspruch 1, dadurch gekennzeichnet, dass das Medium in der der Strahlungsquelle unmittelbar benachbarten Bestrahlungskammer nicht mehr als 50% der in den Durchflussreaktor eintretenden Strahlung absorbiert.

3. Verfahren nach einem der Ansprüche 1 oder 2, dadurch gekennzeichnet, dass dem Medium vor der Bestrahlung ein Oxidationsmittel zugeführt wird.

4. Verfahren nach einem der Ansprüche 1 bis 3, dadurch gekennzeichnet, dass dem Medium während der Bestrahlung ein Oxidationsmittel zugeführt wird.

5. Verfahren nach einem der Ansprüche 1 bis 4, dadurch gekennzeichnet, dass ein Teilstrom des bestrahlten Mediums nach dem Durchlauf in den Durchflussreaktor zurückgeführt wird.

6. Verfahren nach einem der Ansprüche 1 bis 5, dadurch gekennzeichnet, dass das Medium nacheinander durch die Bestrahlungskammern des Durchflussreaktors gefördert wird.

7. Verfahren nach einem der Ansprüche 1 bis 6, dadurch gekennzeichnet, dass das Medium parallel durch die Bestrahlungskammern des Durchflussreaktors gefördert wird.

8. Vorrichtung zur Ausübung des Verfahrens nach Anspruch 1-7, bestehend aus einer Strahlungsquelle mit mindestens einem Strahler, der ultraviolette Strahlung im Wellenlängenbereich von 240 bis 320 nm emittiert, aus einem durch mindestens eine für die ultraviolette Strahlung durchlässige Trennwand in mindestens zwei Bestrahlungskammern unterteilten Durchflussreaktor mit einer Zuleitung und einer Ableitung für das zu bestrahlende Medium, dessen Bestrahlungskammern in bezug auf die durch die Strahlungsquelle bestimmte Durchstrahlungsrichtung hintereinander angeordnet sind, wobei in allen Bestrahlungskammern ein bestimmter Anteil und in der der Strahlungsquelle unmittelbar benachbarten Bestrahlungskammer ein Teil der in den Durchflussreaktor eintretenden ultravioletten Strahlung durch das Medium absorbierbar ist, und aus einer Durchflussteuereinrichtung zur Einstellung eines bestimmten Durchflusses zwecks Einhaltung einer vorbestimmten Mindestdosis der ultravioletten Strahlung, dadurch gekennzeichnet, dass die von der Zahl n der Bestrahlungskammern (8, 9; 23, 39; 23, 49; 109, 110, 111; 209, 211; 309, 311; 509, 511) und von der UV-Durchlässigkeit des zu bestrahlenden Mediums bestimmte Gesamtschichtdicke des Durchflussreaktors (2, 41, 101, 171, 201, 301, 501) so bemessen ist, dass die Gesamtabsorption höchstens im Bereich von $(1-0.5^n) \cdot 100\%$ der in den Durchflussreaktor eintretenden Strahlung liegt.

9. Vorrichtung nach Anspruch 8, dadurch gekennzeichnet, dass die durch die UV-Durchlässigkeit des zu bestrahlenden Mediums bestimmte Schichtdicke der der Strahlungsquelle unmittelbar benachbarten Bestrahlungskammer (8, 39, 49, 109, 209, 311, 509) so bemessen ist, dass die Absorption der UV-Strahlung nicht mehr als 50% der in den Durchflussreaktor (2, 41, 101, 171, 201, 301, 501) eintretenden Strahlung liegt.

10. Vorrichtung nach Anspruch 8 und 9, dadurch gekennzeichnet, dass die Trennwände aus Quarzglas bestehen.

11. Vorrichtung nach Anspruch 8 bis 10 aus einer Strahlungsquelle mit mehreren Strahlern und aus einem Durchflussreaktor mit mehreren Bestrahlungskammern, deren jede einem Strahler zugeordnet ist, dadurch gekennzeichnet, dass jeder nach Art einer Tauchlampe in ein Hüllrohr (25, 45) eingeschlossene Strahler (24) von wenigstens einem Quarzglasrohr (35, 52, 55) umgeben ist und die Strahler (24) in einen gemeinsamen Behälter (21) eingesetzt sind, dass das Hüllrohr (25, 45) und das Quarzglasrohr (35, 55) wenigstens eine innere Bestrahlungskammer (39, 49) begrenzen und dass die inneren Bestrahlungskammern (39, 49) jeweils mit dem Behälterinneren kommunizieren und gemeinsam entweder eingangsseitig an die Zuleitung (91) oder ausgangsseitig an die Ableitung (37, 57, 90) des Durchflussreaktors (41) angeschlossen sind.

12. Vorrichtung nach Anspruch 8 bis 11, bei der die Strahlungsquelle und der Durchflussreaktor ringförmig zueinander angeordnet sind, dadurch gekennzeichnet, dass der Durchflussreaktor (101, 171, 201, 301, 301A; 501) aus zwei mit Anschlussmitteln versehenen Verschlussteilen (103, 104; 173, 174; 203, 204; 303, 304; 303A; 503), die die Bestrahlungskammern (109, 110, 111; 209, 211; 309, 311; 509, 511) stirnseitig begrenzen, und aus zwischen den Verschlussteilen (103, 104; 173, 174; 203, 204; 303, 304; 303A; 503) an diesen angebrachten Rohrstücken (102, 105, 106, 107; 172; 202, 205, 207; 302, 305) unterschiedlichen Durchmessers besteht, die koaxial ineinander angeordnet sind und die Bestrahlungskammern (109, 110, 111; 209, 211; 309, 311; 509, 511) längsseitig begrenzen.

13. Vorrichtung nach Anspruch 12, dadurch gekennzeichnet, dass eine der Strahlungsquelle (24) abgewandte Bestrahlungskammer (111; 211; 309, 509) eine Schichtdicke besitzt, die mindestens das Zweifache der Schichtdicke der der Strahlungsquelle (24) unmittelbar benachbarten Bestrahlungskammer (109; 209; 311; 509) beträgt.

14. Vorrichtung nach Anspruch 11 bis 13, dadurch gekennzeichnet, dass der Durchflussreaktor (41, 101, 201, 301) ausgangsseitig mit einem Strömungsteiler (403, 420) versehen ist, dessen einer Ausgang (423) an die Entnahmeleitung (405) und dessen zweiter Ausgang (422) unter Zwischenschaltung einer Rücklauf-Förderpumpe (409) und eines Rückschlagventils (410) an den Eingang des Durchflussreaktors (41, 101, 201, 301) angeschlossen ist.

15. Vorrichtung nach Anspruch 8 bis 14, dadurch gekennzeichnet, dass die Strahlungsquelle (6, 24) von mindestens einer antimondotierten Xenon-Hochdrucklampe, die eine starke Emission im Wellenlängenbereich von 260 bis 280 nm besitzt, gebildet ist.

16. Vorrichtung nach Anspruch 15, dadurch gekennzeichnet, dass die Strahlungsquelle zusätzlich mindestens eine Quecksilberdampflampe aufweist.

**Patent Claims**

1. Method for purifying a medium capable of flowing, in which said medium is passed through a flow reactor at a certain rate of flow to maintain a predetermined minimum dose of ultraviolet radiation from a radiation source acting on said medium, said flow reactor being subdivided normally with respect to the direction of irradiation by at least one partition transparent with respect to ultraviolet radiation to form at least two irradiation chambers, and in which method a defined proportion of the ultraviolet radiation entering said flow reactor is absorbed by said medium in all the irradiation chambers arranged in series with respect to the direction of irradiation as determined by the radiation source, a portion of said radiation entering said flow reactor becoming absorbed by the medium in that irradiation chamber that is immediately adjacent to said radiation source, characterised in that the medium in all the irradiation chambers does not absorb in total more than $(1-0.5^n) \cdot 100$ percent of the radiation entering the flow reactor, wherein n is the number of irradiation chambers.

2. Method as claimed in Claim 1 characterised in that the medium in the irradiation chamber that is immediately adjacent to the radiation source does not absorb more than 50 percent of the radiation entering the flow reactor.

3. Method as claimed in Claim 1 or Claim 2 characterised in that an oxidising agent is supplied to the medium prior to irradiation.

4. Method as claimed in anyone of Claims 1 to 3 characterised in that an oxidising agent is supplied to the medium during irradiation.

5. Method as claimed in anyone of Claims 1 to 4 characterised in that a portion of the flow of the irradiated medium after having passed through the flow reactor is returned into said flow reactor.

6. Method as claimed in anyone of Claims 1 to 5 characterised in that the medium is passed in series through the irradiation chambers of the flow reactor.

7. Method as claimed in anyone of Claims 1 to 5 characterised in that the medium is passed in parallel through the irradiation chambers of the flow reactor.

8. Apparatus for carrying out the method as claimed in anyone of Claims 1 to 7, said apparatus comprising a radiation source including at least one source of radiation emitting ultraviolet radiation in the wavelength range of 240 nm to 320 nm, a flow reactor including a supply line and a discharge line for the medium to be irradiated and being subdivided by at least one partition transparent with respect to the ultraviolet radiation to form at least two irradiation chambers that are arranged in series with respect to the direction of irradiation as determined by the radiation source, said flow reactor being designed so that a defined proportion of the ultraviolet radiation entering said flow reactor is absorbed by the medium in all the irradiation chambers and a portion of said radiation entering said flow reactor is absorbed in that irradiation chamber that is immediately adjacent to the radiation source, and comprising flow control means for adjusting a certain rate of flow for the medium passing through said flow reactor to maintain a predetermined minimum dose of the ultraviolet radiation acting on said medium, characterised in that the total thickness of the flow reactor (2, 41, 101, 171, 201, 301, 501) as determined by the number (n) of irradiation chambers (8, 9; 23, 29; 23, 49; 109, 110, 111; 209, 211; 309, 311; 509, 511) is dimensioned such that the total absorption at the maximum is in the range of $(1-0.5^n) \cdot 100$ percent of the radiation entering said flow reactor.

9. Apparatus as claimed in Claim 8 characterised in that the thickness of the irradiation chamber (8, 39, 49, 109, 209, 311, 509) that is immediately adjacent to the radiation source as determined by the ultraviolet transparency of the medium to be irradiated is dimensioned such that the absorption of ultraviolet radiation therein amounts to no more than 50 percent of the ultraviolet radiation entering the flow reactor (2, 41, 101, 171, 201, 301, 501).

10. Apparatus as claimed in Claim 8 or Claim 9 characterised in that the partition is made of quartz glass.

11. Apparatus as claimed in anyone of Claims 8 to 10 and comprising a radiation source including a number of individual sources of radiation and a flow reactor having a number of irradiation chambers each of which is associated with a respective one of said individual sources of radiation, characterised in that each individual source of radiation (24) is enclosed in an envelope tube (25, 45) like an immersion-type lamp and is surrounded by at least one tube (35, 52, 55) made of quartz glass, said individual sources of radiation (24) being inserted into a common container (21), that said envelope tube (25, 45) and said tube (35, 55) made of quartz glass define at least one inner irradiation chamber (39, 49) and that said inner irradiation chambers (39, 49) each communicate with the interior of said container and are connected in common either with their inlets to the supply line (91) or with their outlets to the discharge line (37, 57, 90) of the flow reactor (41).

12. Apparatus as claimed in anyone of Claims 8 to 11 with an annular arrangement of the radiation source and the flow reactor relative to each other, characterised in that the flow reactor (101, 171, 201, 301, 301A, 501) comprises two closure members (103, 104; 173, 174; 203, 204; 303, 304; 303A; 503) provided with connecting means and defining the irradiation chambers (109, 110, 111; 209, 211; 309, 311; 509, 511) at their respective end faces and comprises tube members (102, 105, 106, 107; 172; 202, 205, 207; 302, 305) of different diameters mounted to said closure members (103, 104; 173, 174; 203, 204; 303, 304; 303A; 503) and extending in between the same, said tube members being disposed coaxially one within the other and longitudinally defining said irradiation chambers (109, 110, 111; 209, 211; 309, 311; 509, 511).

13. Apparatus as claimed in Claim 12 characterised in that an irradiation chamber (111; 211; 309, 509) remote from the radiation source (24) has a thickness that is at least twice that of the irradiation chamber (109; 209; 311; 511) immediately adjacent to the radiation source (24).

14. Apparatus as claimed in aynone of Claims 11 to 14 characterised in that the flow reactor (41, 101, 201, 301) at its outlet is provided with a flow divider (403, 420) a first output (423) of which is connected to the discharge line (405) and a second output (422) of which is connected to the input of said flow reactor (41, 101, 201, 301) with the interconnection of a return feed pump (409) and a check valve (410).

15. Apparatus as claimed in anyone of Claims 8 to 14 characterised in that the radiation source (6, 24) is formed by at least one antimone-doped high pressure xenon arc having a strong emission in the electromagnetic wavelength range of 260 to 280 nm.

16. Apparatus as claimed in Claim 15 characterised in that the radiation source includes at least one mercury vapour lamp.

**Revendications**

1. Procédé de purification dans lequel, pour maintenir une dose minimale prédéterminée de rayonnement ultraviolet, on refoule un milieu fluide, à un débit déterminé, à travers un réacteur à écoulement subdivisé par au moins une cloison laissant passer les rayons ultraviolets en au moins deux chambres d'irradiation, perpendiculairement à la direction d'irradiation, et dans lequel, dans les chambres d'irradiation disposées en série relativement à la direction d'irradiation définie par la source de rayons, le milieu absorbe dans toutes les chambres d'irradiation une fraction déterminée du rayonnement ultraviolet qui pénètre dans le réacteur à écoulement et, dans la chambre d'irradiation immédiatement voisine de la source de rayons, une partie de ce rayonnement, procédé caractérisé en ce que dans toutes les chambres d'irradiation, le milieu n'absorbe pas au total plus de $(1-0,5^n) \cdot 100\%$ du rayonnement pénétrant dans le réacteur à écoulement, n étant le nombre des chambres d'irradiation.

2. Procédé selon la revendication 1, caractérisé en ce que dans la chambre d'irradiation immédiatement voisine de la source de rayons, le milieu n'absorbe pas plus de 50% du rayonnement pénétrant dans le réacteur à écoulement.

3. Procédé selon l'une quelconque des revendications 1 et 2, caractérisé en ce que l'on amène au milieu un oxydant, avant l'irradiation.

4. Procédé selon l'une quelconque des revendications 1 à 3, caractérisé en ce que l'on amène au milieu un oxydant, pendant l'irradiation.

5. Procédé selon l'une quelconque des revendications 1 à 4, caractérisé en ce que l'on ramène au réacteur à écoulement, après qu'il l'ait traversé, un courant partiel du milieu irradié.

6. Procédé selon l'une quelconque des revendications 1 à 5, caractérisé en ce que l'on refoule successivement le milieu à travers les chambres d'irradiation du réacteur à écoulement.

7. Procédé selon l'une quelconque des revendications 1 à 6, caractérisé en ce que l'on refoule le milieu en parallèle à travers les chambres d'irradiation du réacteur à écoulement.

8. Appareil pour la mise en œuvre du procédé selon les revendications 1 à 7, comprenant une source de rayons, qui comporte au moins un émetteur de rayonnement émettant des rayons ultraviolets d'une longueur d'onde de 240 à 320 nm, un réacteur à écoulement subdivisé par au moins une cloison laissant passer les rayons ultraviolets en au moins deux chambres d'irradiation, muni d'une conduite d'amenée et d'une conduite d'évacuation du milieu à irradier, et dont les chambres d'irradiation sont disposées en série relativement à la direction d'irradiation définie par la source de rayons, le milieu pouvant absorber dans toutes les chambres d'irradiation une fraction déterminée du rayonnement ultraviolet qui pénètre dans le réacteur à écoulement et, dans la chambre d'irradiation immédiatement voisine de la source de rayons, une partie de ce rayonnement, ainsi qu'un dispositif de commande d'écoulement permettant d'établir un débit déterminé, afin de maintenir une dose minimale prédéterminée de rayonnement ultraviolet, appareil caractérisé en ce que l'épaisseur totale de couche du réacteur à écoulement (2, 41, 101, 171, 201, 301, 501), déterminée par le nombre n des chambres d'irradiation (8, 9; 23, 39; 23, 49; 109, 110, 111; 309, 311; 509, 511) et par la transmission de rayons ultraviolets du milieu à irradier, est calculée de façon telle que l'absorption totale soit au maximum de l'ordre de $(1-0,5^n) \cdot 100\%$ du rayon-nement pénétrant dans le réacteur à écoulement.

9. Appareil selon la revendication 8, caractérisé en ce que l'épaisseur de couche de la chambre d'irradiation (8, 39, 49, 109, 209, 311, 509) immédiatement voisine de la source de rayonnement, déterminé par la transmission de rayons ultraviolets du milieu à irradier, est calculée de façon telle que l'absorption de rayons ultraviolets ne dépasse pas 50% du rayonnement pénétrant dans le réacteur à écoulement (2, 41, 101, 171, 201, 301, 501).

10. Appareil selon les revendications 8 ou 9, caractérisé en ce que les cloisons sont formées de verre de quartz.

11. Appareil selon les revendications 8 à 10, comprenant une source de rayons qui comporte plusieurs émetteurs de rayonnement et un réacteur à écoulement qui comporte plusieurs chambres d'irradiation à chacune desquelles est adjoint un émetteur de rayonnement, caractérisé en ce que chaque émetteur de rayonnement (24) enfermé dans un tube d'enveloppe (25, 45) à la manière d'une lampe de plongée, est entouré d'au moins un tube en verre de quartz (35, 52, 55), et les émetteurs de rayonnement (24) sont insérés dans un récipient commun (21), en ce que le tube d'enveloppe (25, 45) et le tube en verre de quartz (35, 55) délimitent au moins une chambre d'irradiation intérieure (39, 49) et en ce que les chambres d'irradiation intérieures (39, 49) communiquent chacune avec l'intérieur du récipient et sont raccordées conjointement, soit du côté de l'entrée à la conduite d'amenée (91), soit du côté de la sortie à la conduite d'évacuation (37, 57, 90) du réacteur à écoulement (41).

12. Appareil selon les revendications 8 à 11, dans lequel la source de rayons et le réacteur à écoulement ont une disposition relative annulaire, caractérisé en ce que le réacteur à écoulement (101, 171, 201, 301, 301A; 501) est formé de deux éléments de fermeture (103, 104; 173, 174; 203, 204; 303, 304; 303A; 503) munis de moyens de raccordement, qui limitent du côté frontal les chambres d'irradiation (109, 110, 111; 209, 211; 309, 311; 509, 511) et de tronçons de tube (102, 105, 106, 107; 172; 202, 205, 207; 302, 305) de diamètre différent, adjoints aux éléments de fermeture (103, 104; 173, 174; 203, 204; 303, 304; 303A; 503), disposés entre ceux-ci, placés coaxialement l'un dans l'autre et limitant longitudinalement les chambres d'irradiation (109, 110, 111; 209, 211; 309, 311; 509, 511).

13. Appareil selon la revendication 12, caractérisé en ce qu'une chambre d'irradiation (111; 211; 309, 509) opposée à la source de rayons (24) présente une épaisseur de couche au moins double de l'épaisseur de couche de la chambre d'irradiation (109; 209; 311; 511) immédiatement voisine de la source de rayons (24).

14. Appareil selon les revendications 11 à 13, caractérisé en ce que le réacteur à écoulement (41, 101, 201, 301) est muni du côté de la sortie d'un diviseur d'écoulement (403, 420) dont l'une des sorties (423) est raccordée à la conduite de retrait (405) et dont la deuxième sortie (422) est raccordée, avec interposition d'une pompe de reflux (409) et d'une valve antiretour (410), à l'entrée du réacteur à écoulement (41, 101, 201, 301).

15. Appareil selon les revendications 8 à 14, caractérisé en ce que la source de rayons (6, 24) est formée d'au moins une lampe à xenon à haute pression dopée à l'antimoine, qui présente une forte émission dans la gamme de longueurs d'onde de 260 à 280 nm.

16. Appareil selon la revendication 15, caractérisé en ce que la source de rayons comporte, en outre, au moins une lampe à vapeur de mercure.

FIG. 1

0 000 773

FIG. 3

FIG. 2

FIG.4

FIG.5

48
46
48
45
57
24
55
49
68
67 27 28 71

72
71
70
70
69
69
70
69

29
67
30

33

FIG.4A

FIG.2A

FIG.6

FIG. 7

FIG. 8

FIG. 9

FIG. 10

FIG. 11

FIG. 12

FIG.13

FIG.14

FIG. 13 A

FIG.15

0 000 773

FIG.16

FIG. 17

329
356
357 354
328
301A
127
133 303A
326
325
309
305
324
323
358 321
320 319 318
355 312
322 313
311 316
302 317

57

0 000 773